# EUROPEAN PATENT SPECIFICATION

(11) **EP 3 679 649 B1**
(45) Date of publication and mention of the grant of the patent: **09.06.2021**
(21) Application number: 18796102.4
(22) Date of filing: 05.09.2018
(51) Int. Cl.: H03F 3/193, H01L 29/423, A61B 5/05, A61B 5/145, H01L 31/112, A61B 5/00, A61B 5/1172, A61B 5/117

(54) **MICROELECTRONIC SENSOR FOR BIOMETRIC AUTHENTICATION**
MIKROELEKTRONISCHER SENSOR ZUR BIOMETRISCHEN AUTHENTIFIZIERUNG
CAPTEUR MICROÉLECTRONIQUE POUR AUTHENTIFICATION BIOMÉTRIQUE

(30) Priority: 05.09.2017 US 201762554098 P
(43) Date of publication of application: 15.07.2020
(73) Proprietor: Epitronic Holdings Pte. Ltd., Singapore 079027 (SG)
(72) Inventor: RAM, Ayal, Singapore 079027 (SG); MUNIEF, Walid-Madhat, 66440 Blieskastel (DE)
(74) Representative: Lavoix
(86) International application number: PCT/IB2018/056765
(87) International publication number: WO 2019/049035

(56) References cited:
- HAOWEN HOU ET AL: "A sub-terahertz broadband detector based on a GaN high-electron-mobility transistor with nanoantennas", APPLIED PHYSICS EXPRESS IOP PUBLISHING UK, vol. 10, no. 1, January 2017 (2017-01), XP002787686, ISSN: 1882-0778
- SOKOLOVSKIJ R ET AL: "Precision Recess of AlGaN/GaN with Controllable Etching Rate Using ICP-RIE Oxidation and Wet Etching", PROCEDIA ENGINEERING, ELSEVIER, AMSTERDAM, NL, vol. 168, 4 January 2017 (2017-01-04), pages 1094-1097, XP029874885, ISSN: 1877-7058, DOI: 10.1016/J.PROENG.2016.11.350

## Description

### TECHNICAL FIELD

The present application relates to the field of microelectronic sensors based on high-electron-mobility transistors and their use in detection and continuous monitoring of electrical signals generated by a human body in a sub-terahertz range. In particular, the present application relates to the open-gate pseudo-conducting high-electron-mobility transistors (PC-HEMTs) and their use in biometric authentication.

### BACKGROUND

There are many different types of biometric authentication systems nowadays used in smartphones, key locks, airport and high security locations where an extremely high level of authentication is required. Commercially available authentication systems may include a fingerprint ID sensor, an iris ID sensor or speech recognition. However, these systems proved to have a relatively low security level.

In 2014, a new type of the biometric authentication device by Bionym, based on the electrocardiogram (ECG), has been introduced on the market. This device, called "Nymi", is capable of capturing a unique electrocardiographic waveform of a person, and further maps it to ECG patterns. Nymi is continuously worn as a bracelet or wristband on the user's wrist and uses learning algorithms to memorise the user's ECG patterns in order to increase the quality and authentication level of the user. WO 2012151680 by Bionym discloses a biometric sensor based on the analysis of the ECG signals used to authenticate one or more individuals. This sensor relies on authenticating identity by matching the overall shape of the user's ECG waveform (captured via an electrocardiogram sensor).

Unlike other authentication methods, such as fingerprint scanning and iris-recognition, the sensor by Bionym sustains authentication so long as the wearer keeps the wristband on. To authenticate via Nymi, the user puts the wristband on and touches a topside sensor with one hand to complete an electrical loop with the bottom-side sensor touching their wrist. That generates the ECG data used to authenticate their identity, and the wristband transmits the ECG via Bluetooth to the corresponding registered app, on a smartphone or other external device in proximity to the user, to verify the wearer's identity.

The biometric authentication devices, such as Nymi, and similar ECG-based devices however have several drawbacks. First, they use a single data source (ECG) for mapping the users' unique ID profiles, making them a prime target for signal cloning and similar security-breaking techniques. Second, these devices are not closed systems and cannot provide a single closed integrated chip solution, which can be considered another tampering and security-breaching issue on a hardware level. Nymi needs to be worn at all times, in order to use the authentication system, which may be intrusive to some users.

In addition, Nymi and similar ECG-based devices do not provide a single-contact point for simple authenticate of a user, and require the user to perform rather complex steps and procedures to authenticate themselves prior to each use, for example to use both hands to close a circuit and authenticate the user, the process each user must perform on a daily basis. Therefore, these technologies cannot be integrated directly into end-point devices, such as credit cards and mobile phones, for a single-point authentication.

However, there are two major disadvantages of using the above ECG-based sensors. First, many medications and various psychological conditions can directly affect, change and alter the heart rhythmic cycles, which are recorded by the sensor, so that the general authentication system may fail at times. Second, the ECG-based sensor or similar sensor sensing the hemodynamic electrical signals of the heart and lungs requires a full heart cycle or even two in order to authenticate the user. In a real-world application, this is a huge drawback in comparison to the existing capacitive fingerprint sensors, since it would take too long to authenticate the user or, for example to unlock a phone, compared to existing one-second authentication time.

A high-electron mobility transistor is known from HAOWEN HOU ET AL: "A sub-terahertz broadband detector based on a GaN high-electron-mobility transistor with nanoantennas", APPLIED PHYSICS EXPRESS, vol. 10, no. 1.

In view of the above, there is a long-felt need to develop a new biometric authentication sensor based on recording the user's physiological signals, which would overcome the aforementioned drawbacks of the existing ECG-based sensors measuring the heart dipole cycle.

Recently it has become clear that terahertz (THz) radiation could be extremely important for research related to the life sciences because of the unique capability of these low energy electromagnetic waves to interact with vibrations of atoms within biological molecules to produce specific molecular fingerprints (see for example, Globus et al. in "Terahertz Fourier transform characterization of biological materials in a liquid phase", J. Physics D: Applied Physics, 39(15), 3405-3413). Sub-THz spectroscopy uses wavelengths beyond those traditionally used for chemical and biomolecular analysis. Biological materials have found to be active in the frequency range of 0.05-1 THz and above (the submillimetre-wavelength range, about 1.5 to 30 cm⁻¹). These frequency and wavelength domains, the spectral range between the upper end of the radio frequencies and the lowest optical frequencies were named the 'Terahertz Gap', because so little was known about them and because of the absence of radiation sources and detectors.

Sub-THz vibrational spectroscopy is based on the interaction of THz radiation with internal molecular vibrations of low energy. A majority of the THz experimental data have recently been reported on frequencies above 1 THz and for relatively small biological molecules that are often prepared in crystalline form (for example, Heilweil et al. (2008), "Terahertz Spectroscopy of Biomolecules", In Terahertz Spectroscopy, Taylor and Francis, London, 2008, Chapter 7, pp 269-297). Low energy THz radiation interacts with the low-frequency internal molecular motions (vibrations) involving the weakest hydrogen bonds (H-bonds) and other weak connections within molecules by exciting these vibrations. The width of individual spectral lines and the intensity of resonance features, which are observed in sub-THz spectroscopy, are very sensitive to the relaxation processes of atomic dynamics (displacements) within a molecule. Those relaxation processes determine the discriminative capabilities of sub-THz spectroscopy. Appropriate spectral resolution must be used in THz spectroscopy to be able to acquire qualitative as well as quantitative information used to identify the molecules that will, in turn, increase detection accuracy and selectivity.

Because of their small size and relatively low absorption coefficient, the waves of the THz radiation easily propagate through the entire biological object, such as cells and skin. Safrai et al. (2012) in "The remote sensing of mental stress from the electromagnetic reflection coefficient of human skin in the sub-THz range", Bioelectro-magnetics, 2012, 33(5), 375-82, and Safrai et al. (2014-1) in "Remote monitoring of phasic heart rate changes from the palm" in IEEE Transactions on Terahertz Science and Technology, 2014, 4, 618-624, reported that both physical and mental stress could be traced through the reflection coefficient of the hand, influenced by the activity of the sweat ducts, in the (75 GHz-110 GHz) and (110 GHz-170 GHz) frequency bands. The reflected signal was monitored from a distance of 72 cm using a Vector Network Analyser, while the patients' electrocardiograms (ECGs) were concurrently registered. Further, Safrai et al. (2014-2) in "The correlation of ECG parameters to the sub-THz reflection coefficient of human skin", IEEE Transactions on Terahertz Science and Technology, 2014, 4(5), 624-630, reported on a good correlation between the reflection coefficient in the same frequency bands and some of the parameters of the ECG, mainly to the ST elevation.

### Sweat Ducts as Helical Antennas

Hayut et al. (2013) in "The helical structure of sweat ducts: Their influence on the electromagnetic reflection spectrum of the skin", IEEE Transactions on Terahertz Science and Technology, 2013, Vol. 3, No. 2, pp. 2017-2015, suggested that the helical structure of human eccrine sweat ducts together with the dielectric properties of the human skin would result in their electromagnetic properties similar to those of an array of helical antennas. In order to test this assumption, they conducted numerical simulations in the sub-THz frequency range of 100-450 GHz, performed measurements of a reflection spectrum from the human skin and compared the obtained results to the simulation results.

The spectral response obtained by their simulations coincided with the analytical prediction of antenna theory and supported the hypothesis that the sweat ducts can indeed be regarded as helical antennas. The magnitude of the spectral response was found to be dependent on the conductivity of sweat in these frequencies, but the analysis of the frequencies related to the antenna-like modes were found to be independent of this parameter. The performed simulations demonstrate that variations of the spectra are observed in the vicinity of the frequencies close to the predicted axial response mode of the sweat ducts (regarded as helical antennas) at approximately 380 GHz. The results clearly show that the structure of the sweat ducts plays a key role in the shaping of the reflected spectra. Thus, it has been established that the coiled nature of the human sweat duct can lead to electromagnetic behaviour reminiscent of a helical antenna.

In view of the above, the present inventors proposed that analysis of sweat using electrochemical devices on human skin may provide a new route for biometric authentication. Every individual has his/her own sub-THz spectra and his/her own sweat-duct spatial skin-surface pattern. Using this unique sub-THz-pattern, it might be possible to record the very unique spectra and spatial duct pattern from each individual and then to authenticate them with an unprecedented precision.

### SUMMARY

The present application describes embodiments of a microelectronic sensor based on a combination of an open-gate pseudo-conducting high-electron mobility transistor (PC-HEMT) and Vivaldi antenna. In some embodiments, the transistor comprises a substrate, on which a multilayer heterojunction structure is deposited. This hetero-junction structure may comprise at least one buffer layer and at least one barrier layer grown from III-V single-crystalline or polycrystalline semiconductor materials and stacked alternately. In some embodiments, the III-V single-crystalline or polycrystalline semiconductor materials are GaN/AlGaN.

A conducting channel comprising a two-dimensional electron gas (2DEG) or a two-dimensional hole gas (2DHG) is formed at the interface between the buffer and barrier layers and upon applying a bias to said transistor provides electron or hole current in the transistor between its source and drain electrodes. In a particular embodiment, the heterojunction structure may be a three-layer structure consisting of two GaN layers and one AlGaN layer squeezed between said GaN layers like in a sandwich. This may lead to formation of the two-dimensional electron gas (2DEG) or two-dimensional hole gas (2DHG) in the top layer dependent on the polarity of the structure.

An optional dielectric layer may be deposited on top of the heterojunction structure. The open gate area of the 2DEG/2DHG is formed between the source and drain areas as a result of recessing or growing of the top layer to a specific thickness.

The source and drain non-ohmic (i.e. capacitively-coupled) contacts are connected to the 2DEG/2DHG channel and to electrical metallizations, the latter are placed on top of the sensor and connect it to an electric circuit of the sensor. Since the source and drain contacts are non-ohmic, the DC readout cannot be carried out. Instead, in order to electrically contact the 2DEG/2DHG channel underneath, about 5-20 nm bellow the metallizations, the AC readout or impedance measurements of the electric current flowing through the 2DEG/2DHG-channel must be performed. In this case, the capacitive coupling of the non-ohmic metal contacts with the 2DEG/2DHG channel is normally induced at the frequency higher than 30 kHz.

In some embodiments, the PC-HEMT multilayer heterojunction structure of the embodiments is grown from any available III-V single-crystalline or polycrystalline semiconductor materials, such as GaN/AlGaN, GaN/AIN, GaN/InN, GaN/InAlGaN, GaAs/AlGaAs, GaN/InAIN, InN/InAIN or LaAlO₃/SrTiO₃. In a particular case of the substrate grown from GaN/AlGaN, it has been experimentally and surprisingly found that the highest sensitivity of the sensor is achieved when thickness of the top recessed layer (GaN buffer layer or AlGaN barrier layer) in the open gate area between the source and drain contacts is 5-9 nm, preferably 6-7 nm, more preferably 6.2-6.4 nm. This recessed layer thickness corresponds to the pseudo-conducting current range between normally-on and normally-off operation mode of the 2DEG/2DHG conducting channel. In addition, surface roughness of the top recessed layer within the open gate area between the source and drain contacts has a roughness of about 0.2 nm or less, preferably 0.1 nm or less, more preferably 0.05 nm. Thus, the significant features of the PC-HEMT are that:
(i) thickness of the top recessed layer (GaN layer or AlGaN layer) of the multilayer heterojunction structure in the open gate area between the source and drain contacts is 5-9 nm, preferably 6-7 nm, more preferably 6.2-6.4 nm, which corresponds to the pseudo-conducting current range between normally-on and normally-off operation mode of the 2DEG/2DHG conducting channel;
(ii) surface roughness of the top recessed layer in the open gate area between the source and drain contacts is about 0.2 nm or less, preferably 0.1 nm or less, more preferably 0.05 nm; and
(iii) the non-ohmic source and drain contacts for the capacitive coupling with the conductive 2DEG/2DHG channel replace the ohmic contacts.

In a further embodiment, the present application provides the sensing device suitable for use in biometric authentication.

Various embodiments may allow various benefits, and may be used in conjunction with various applications. The details of one or more embodiments are set forth in the accompanying figures and the description below. Other features, objects and advantages of the described techniques will be apparent from the description and drawings and from the claims

### BRIEF DESCRIPTION OF THE DRAWINGS

Disclosed embodiments will be understood and appreciated more fully from the following detailed description taken in conjunction with the appended figures. The drawings included and described herein are schematic and are not limiting the scope of the disclosure. It is also noted that in the drawings, the size of some elements may be exaggerated and, therefore, not drawn to scale for illustrative purposes. The dimensions and the relative dimensions do not necessarily correspond to actual reductions to practice of the disclosure.
**Fig. 1** schematically shows the quantum well at three different biasing conditions:
   **Fig. 1a****:** positive gate potential (+V_{G}) is much higher than threshold voltage (V_{T}),
   **Fig. 1b****:** 0V gate potential, and
   **Fig. Ic:** negative gate potential (-V_{G}) is below threshold voltage (V_{T}).
**Fig.** 2 schematically shows a cross-sectional (XZ) view (A-A) of the PC-HEMT of an embodiment.
**Fig. 3** schematically shows the top (XY) view and the basic topology of the sensor of an embodiment.
**Fig. 4a** schematically shows the dependence of the source-drain current (a charge carrier density) induced inside the 2DEG channel of a GaN/AlGaN HEMT on the thickness of the AlGaN layer recessed in the open gate area.
**Fig. 4b** illustrates a theory behind the 2DEG formation (charge neutrality combined with the lowest energy level) at the conduction band discontinuity.
**Fig. 5a** schematically shows the 2DEG area created in the step of the 2DEG-pattering via ion implantation during the manufacturing process. AZ 4533 is a positive thick resist.
**Fig. 5b** shows the lithographic mask of the sensor layout of the present invention.
**Fig. 5c** shows the lithographic image of the 2DEG channel formed with AZ 4533 thick resist lithography over the mask shown in **Fig. 5b****.**
**Figs. 5d-5e** show the mask and the corresponding lithographic image, respectively, of the sensor layout of the present invention.
**Fig. 5f** shows the ± 2-µm alignment precision on 25 x 25 mm² samples in the lithography of the sensor layout of the present invention.
**Fig. 5g** shows the lithographic images of the multichannel samples.
**Fig. 5h** shows the fixed sample on the Si-GaN/AlGaN wafer prepared for ion implantation and containing around 30-32 sensors with 4-8 channels on each sample.
**Fig. 5i** shows the lithographic image of the sensor layout with the AZ4533 resist after development, prepared for ion implantation.
**Fig. 5j** shows the 2DEG channels (dark) patterned by ion-implantation after the resist removal.
**Fig. 5k** shows the visible non-implanted area containing the conductive 2DEG channel.
**Fig. 6a** shows the AFM surface image of the top recessed layer of the PC-HEMT made by the manufacturing process of the present invention. The measured RMS value of the surface roughness is 0.674 nm in this case.
**Fig. 6b** shows the AFM surface image of the top recessed layer of the HEMT made by a conventional manufacturing process. The measured RMS value of the surface roughness is 1.211 nm in this case.
**Fig. 6c** shows the time-dependent plot of the drain-source electric current I_{DS} of the nitrogen oxide sensor measuring 100 ppb of the NO₂ gas in humid air, where the sensor is based on the PC-HEMT made by the manufacturing process of the present invention.
**Fig. 6d** shows the time-dependent plot of the drain-source electric current I_{DS} of the nitrogen oxide sensor measuring 100 ppb of the NO₂ gas in humid air, where the sensor is based on the HEMT made by a conventional manufacturing process.
**Fig. 7a** schematically shows the formation of the 2DEG and 2DHG conducting channels in the Ga-face three-layer Ga/AlGaN/GaN PC-HEMT structure.
**Fig. 7b** schematically shows the formation of the 2DEG and 2DHG conducting channels in the N-face three-layer Ga/AlGaN/GaN PC-HEMT structure.
**Fig. 8** schematically shows the formation of the 2DEG conducting channel in the N-face three-layer GaN/AlGaN/GaN PC-HEMT structure with an ultrathin Al(GaN)N layer for improved confinement.
**Fig. 9** shows the model of a Vivaldi antenna realised on a thin dielectric substrate. An exponential function is used for the taper profile. The entire domain is bounded by a perfectly matched layer.
**Fig. 10** shows the electric field distribution in the Vivaldi antenna plane at 480 GHz.
**Fig. 11** shows (a) the far-field directional radiation pattern of the Vivaldi antenna starting from 240 GHz till 780 GHz and (b) the corresponding 3D far-field pattern at 480 GHz.
**Fig. 12** shows the exemplary two-dimensional photonic crystals (a = 160 µm, d = 60 µm) deposited on top of the metal layer of the Vivaldi antenna.
**Fig. 13** shows the position of the metal connector on the Vivaldi antenna for coupling with the PC-HEMT of an embodiment.
**Fig. 14** schematically shows a microelectronic sensor comprising a single PC-HEMT of the embodiments with the integrated Vivaldi antenna, for non-invasive monitoring of glucose levels in blood, with a remote readout.
**Fig. 15** schematically shows a microelectronic sensor comprising an array of the PC-HEMTs of the embodiments with the integrated Vivaldi antennas, for non-invasive monitoring of glucose levels in blood, with a remote readout.

### DETAILED DESCRIPTION

In the following description, various aspects of the present application will be described. For purposes of explanation, specific configurations and details are set forth in order to provide a thorough understanding of the present application. However, it will also be apparent to one skilled in the art that the present application may be practiced without the specific details presented herein. Furthermore, well-known features may be omitted or simplified in order not to obscure the present application.

The term "comprising", used in the claims, is "open ended" and means the elements recited, or their equivalent in structure or function, plus any other element or elements which are not recited. It should not be interpreted as being restricted to the means listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a device comprising x and z" should not be limited to devices consisting only of components x and z. Also, the scope of the expression "a method comprising the steps x and z" should not be limited to methods consisting only of these steps.

Unless specifically stated, as used herein, the term "about" is understood as within a range of normal tolerance in the art, for example within two standard deviations of the mean. In one embodiment, the term "about" means within 10% of the reported numerical value of the number with which it is being used, preferably within 5% of the reported numerical value. For example, the term "about" can be immediately understood as within 10%, 9%, 8%, 7%, 6%, 5%, 4%, 3%, 2%, 1%, 0.5%, 0.1%, 0.05%, or 0.01% of the stated value. In other embodiments, the term "about" can mean a higher tolerance of variation depending on for instance the experimental technique used. Said variations of a specified value are understood by the skilled person and are within the context of the present invention. As an illustration, a numerical range of "about 1 to about 5" should be interpreted to include not only the explicitly recited values of about 1 to about 5, but also include individual values and sub-ranges within the indicated range. Thus, included in this numerical range are individual values such as 2, 3, and 4 and sub-ranges, for example from 1-3, from 2-4, and from 3-5, as well as 1, 2, 3, 4, 5, or 6, individually. This same principle applies to ranges reciting only one numerical value as a minimum or a maximum. Unless otherwise clear from context, all numerical values provided herein are modified by the term "about". Other similar terms, such as "substantially", "generally", "up to" and the like are to be construed as modifying a term or value such that it is not an absolute. Such terms will be defined by the circumstances and the terms that they modify as those terms are understood by those of skilled in the art. This includes, at very least, the degree of expected experimental error, technical error and instrumental error for a given experiment, technique or an instrument used to measure a value.

As used herein, the term "and/or" includes any and all combinations of one or more of the associated listed items. Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and relevant art and should not be interpreted in an idealized or overly formal sense unless expressly so defined herein. Well-known functions or constructions may not be described in detail for brevity and/or clarity.

It will be understood that when an element is referred to as being "on", "attached to", "connected to", "coupled with", "contacting", etc., another element, it can be directly on, attached to, connected to, coupled with or contacting the other element or intervening elements may also be present. In contrast, when an element is referred to as being, for example, "directly on", "directly attached to", "directly connected to", "directly coupled" with or "directly contacting" another element, there are no intervening elements present. It will also be appreciated by those of skill in the art that references to a structure or feature that is disposed "adjacent" another feature may have portions that overlap or underlie the adjacent feature.

The polarization doped high-electron-mobility transistor (HEMT) is a field effect transistor (FET) in which two layers of different bandgap and polarisation field are grown upon each other forming the heterojunction structure. This transistor is essentially based on at least two layers of III-V semiconductor materials, such as gallium nitride (GaN) and aluminium gallium nitride (AlGaN). As a consequence of the discontinuity in the polarisation field, surface charges are created at the interface between the layers. If the induced surface charge is positive, electrons will tend to compensate the induced charge resulting in the formation of the channel. Since the channel electrons are confined in a quantum well in an infinitely narrow spatial region at the interface between the layers, these electrons are referred to as a two-dimensional electron gas (2DEG). This special confinement of the channel electrons in the quantum well actually grants them two-dimensional features, which strongly enhance their mobility surpassing the bulk mobility of the material in which the electrons are flowing.

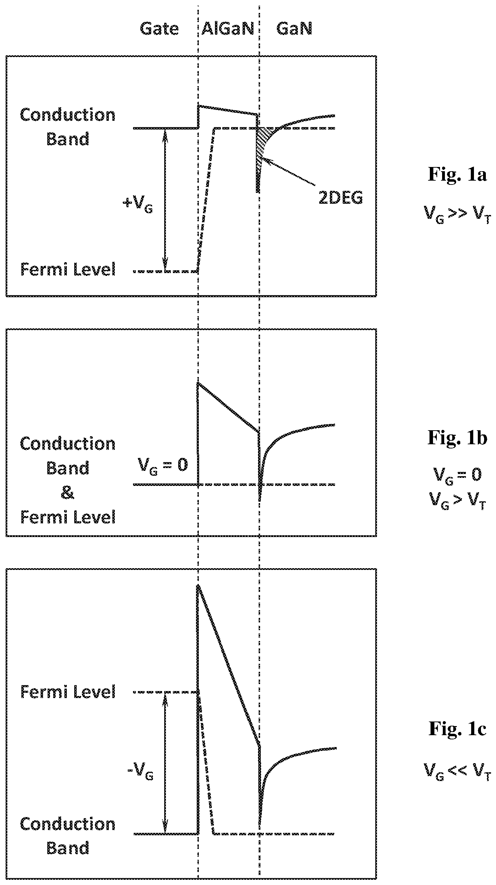

**Figs. 1a-1c** schematically shows the quantum well at three different biasing conditions starting from the positive gate potential (V_{G}), much higher than the threshold voltage (V_{T}), and going down to the 0V gate potential and further to the negative values below the threshold voltage. The V_{T} is defined as a voltage, which is required to populate electrons at the interface between the GaN layer and the AlGaN layers, thereby creating conductivity of the 2DEG channel. Since the 2DEG channel electrons occupy energy levels below the Fermi level, the Fermi level in a quantum well is located above several energy levels when V_{G} >> V_{T} **(****Fig. 1a****).** This enables high population of channel electrons and consequently, high conductivity. The 2DEG channel is turned on in this case. However, when V_{G} decreases to 0V **(****Fig. 1b****),** the Fermi level also drops with respect to the quantum well. As a result, much fewer electron energy levels are populated and the amount of the 2DEG channel electrons significantly decreases. When V_{G} much less than V_{T} **(****Fig. 1c****),** all electron energy levels are above the Fermi level, and there is no the 2DEG electrons below the gate. This situation is called "channel depletion", and the channel is turned off.

Many commercially available HEMTs based on the layers of III-V semi-conductor materials have a negative value of V_{T}, resulting in a "normally-on" operation mode at 0V gate potential. They are called "depletion-mode" semiconductor transistors and used in various power switching applications when the negative voltage must be applied on the gate in order to block the current. However, for safe operation at high voltage or high power density, in order to reduce the circuit complexity and eliminate standby power consumption, the transistors with "normally-off" characteristics are preferred. The high voltages and high switching speeds allow smaller, more efficient devices, such as home appliances, communications and automobiles to be manufactured. To control the density of electrons in the 2DEG channel and to switch the HEMT on and off, the voltage at the gate of the transistor is normally regulated.

Several techniques to manufacture the normally-off semiconductor structures have been reported. Burnham *et al* (2010) proposed normally-off structures of the recessed gate type. In this structure, the AlGaN barrier layer is etched and the gate is brought closer to the interface between the AlGaN barrier layer and the GaN buffer layer. As the gate approaches the interface between the layers, the V_{T} increases. Thus, the normally-off operation of the 2DEG conducting channel is achieved once the depletion region reaches the interface and depletes the 2DEG channel at zero gate voltage. The major advantages of these structures are relatively lower power consumption, lower noise and simpler drive circuits. They are currently used, for example, in microwave and millimetre wave communications, imaging and radars.

Chang *et al* (2009) proposed instead of etching the relatively thick barrier layer to approach the AlGaN/GaN interface, to use a very thin AlGaN barrier. This structure also achieves the normally-off operation of the 2DEG channel by approaching the gate towards the AlGaN/GaN interface. Chen *et al* (2010) proposed to use the fluorine-based plasma treatment method. Although many publications have adopted various methods to achieve normally-off devices with minimum impact on the drain current, they unfortunately sacrificed device turn-on performance.

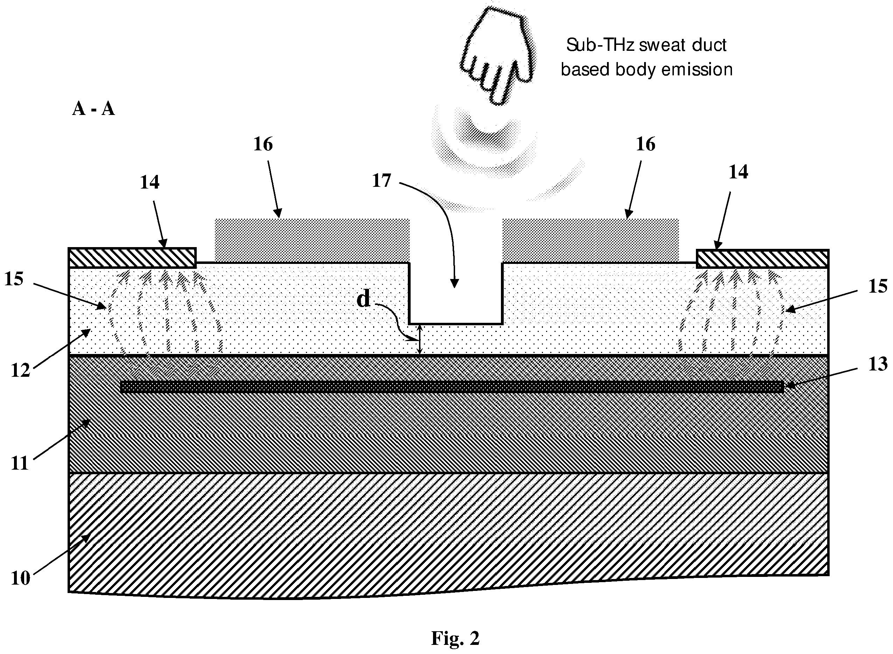

The present application describes embodiments of a microelectronic sensor based on a combination of an open-gate pseudo-conducting high-electron mobility transistor (PC-HEMT) and Vivaldi antenna installed in the open gate area of the transistor. As shown in **Fig. 2****,** the PC-HEMT of the present application, capable of sensing sub-THz radiation produced by a body of the user, comprises:
(a) a multilayer heterojunction structure composed of III-V single-crystalline or polycrystalline semiconductor materials, said structure comprising at least one buffer layer (11) and at least one barrier layer (12), said layers being stacked alternately, and said structure being deposited on a substrate layer (10);
(b) a conducting channel (13) comprising a two-dimensional electron gas (2DEG) or a two-dimensional hole gas (2DHG), formed at the interface between said buffer layer (11) and said barrier layer (12), and upon applying a bias to said transistor, capable of providing electron or hole current in the transistor between source and drain contact areas (15);
(c) electrical metallizations (14) capacitively-coupled to said 2DEG or 2DHG conducting channel (13) via the source and drain contact areas (15) for connecting said transistor to an electric circuit; and
(d) a Vivaldi antenna electrode (16) placed on a top layer between said source and drain contact areas (15) in an open gate area of the transistor and capable of detecting electrical signals in the sub-THz-frequency range of 200-800 GHz.

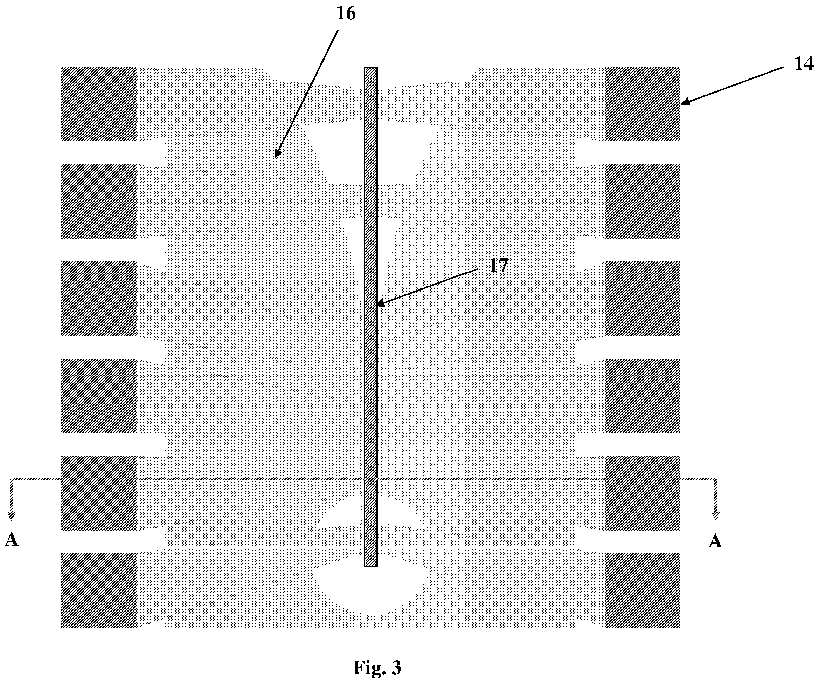

The functional basic topology of the microelectronic sensor of the present embodiments is schematically shown in **Fig. 3****.** The sensor detection principle is based on the field effect of electric current modulation in a DC-mode within the 2DEG/2DHG conducting channel (13) achieved by Vivaldi-shaped gate antenna (16). In fact, the Vivaldi-shaped gate antenna (16) is capable of strongly concentrating the electric field strength of sub-THz radiation in a very small area above the ultra charge-sensitive 2DEG/2DHG channel, thereby affecting its conductivity in the DC-mode by accumulation effect. The S11-S12 parameters of the PC-HEMT of the present embodiments however may also be measured at radio frequencies (RF) of 1 to 60 GHz using the 'beating' effect of sub-THz radiation.

The transistor shown in **Figs. 2** and **3** may further comprise a dielectric layer of 1-10 nm thickness. This dielectric layer may be deposited on top of the barrier layer (12). The Vivaldi antenna gate electrode, made for example from gold, is then placed directly on the dielectric layer. This configuration prevents strong electrical leakage at the metal/top layer interface. The dielectric layer used for the device passivation, may be made, for example, of SiO-SiN-SiO ("ONO") stack of 100-100-100 nm thickness or SiN-SiO-SiN ("NON") stack having the same thicknesses. It may be deposited on top of the barrier layer by a method of plasma-enhanced chemical vapour deposition (PECVD), which is a stress-free deposition technique.

The electrical metallizations (14) connect the transistor to the electric circuit and allow the electric current to flow between non-ohmic contact areas (15) via the two-dimensional electron gas (2DEG) or two-dimensional hole gas (2DHG) channel (13). The metallizations (14) may be made of metal stacks, such as Cr/Au, Ti/Au, Ti/W, Cr/Al and Ti/Al. The Cr or Ti layers of the metal stack is, for example, of 5-10 nm thickness, while the second metal layer, such as Au, W and Al, is of 100-400 nm thickness. The metallizations (14) may be chosen according to the established technology and assembly line at a particular clean room fabrication facility.

In some embodiments, substrate layer (10) may be composed, for example, of sapphire, silicon, silicon carbide, gallium nitride or aluminium nitride. The hetero-junction structure (11, 12) may be deposited on the substrate layer (10), for example, by a method of metalorganic chemical vapour deposition (MOCVD), thereby forming the pseudo-conducting 2DEG or 2DHG channel (13) in the close proximity to the interface between the buffer layer (11) and the barrier layer (12). The barrier layer (12) then may be either recessed or grown in the recessed area (17) as a very thin layer between the source and drain contact areas (15).

The 2DEG or 2DHG channel (13) formed near the interface between the buffer layer (11) and the barrier layer (12) serves as a main sensitive element of the transistor reacting to a surface charge and potential. The 2DEG or 2DHG channel (13) is configured to interact with very small variations in surface or proximal charge or changes of electrical field on the top layer/Vivaldi antenna gate interface interacting with the donor-like surface trap states of the barrier layer. This will be discussed below in detail.

The term "2DEG" mentioned in the present description and claims should not be understood or interpreted as being restricted to the two-dimensional electron gas. As stated above and will be explained later in this application, the two-dimensional hole gas may also be a possible current carrier in a specific heterojunction structure. Therefore, the term "2DEG" may be equally replaced with the term "2DHG" without reference to any particular PC-HEMT configuration.

In some embodiments, the PC-HEMT multilayer heterojunction structure of the embodiments is grown from any available III-V single-crystalline or polycrystalline semiconductor materials, such as GaN/AlGaN, GaN/AIN, GaN/InAlGaN, GaN/InAIN, GaN/InN, GaAs/AlGaAs, InN/InAIN or LaAlO₃/SrTiO₃. In a specific case of the hetero-junction structure grown from GaN/AlGaN, it has been experimentally found that the highest sensitivity of the sensor is achieved when thickness of the top recessed layer (GaN or AlGaN) in the open gate area between the source and drain contacts is 5-9 nm, preferably 6-7 nm, more preferably 6.2-6.4 nm. In addition, it was also found that the sensor exhibits its highest sensitivity when surface roughness of the top recessed layer is about 0.2 nm or less, preferably 0.1 nm or less, more preferably 0.05 nm.

Thus, the top layer recessed in the open gate area to 5-9 nm must be optimised for significantly enhancing sensitivity of the sensor. This specific thickness of the top layer was surprisingly found to correspond to the "pseudo-conducting" current range between normally-on and normally-off operation modes of the 2DEG channel and requires further explanation.

"Pseudo-conducting" (to distinguish from normally-conducting) current range of the 2DEG channel is defined as an operation range of the channel between its normally-on and normally-off operation modes. "Trap states" are states in the band-gap of a semiconductor which trap a carrier until it recombines. "Surface states" are states caused by surface reconstruction of the local crystal due to surface tension caused by some crystal defects, dislocations, or the presence of impurities. Such surface reconstruction often creates "surface trap states" corresponding to a surface recombination velocity.

Classification of the surface trap states depends on the relative position of their energy level inside the band gap. The surface trap states with energy above the Fermi level are acceptor-like, attaining negative charge when occupied. However, the surface trap states with energy below the Fermi level are donor-like, positively charged when empty and neutral when occupied. These donor-like surface trap states are considered to be the source of electrons in the formation of the 2DEG channel. They may possess a wide distribution of ionization energies within the band gap and are caused by redox reactions, dangling bonds and vacancies in the surface layer. A balance always exists between the 2DEG channel density and the number of ionised surface donors which is governed by charge neutrality and continuity of the electric field at the interfaces.

Thus, the donor-like surface traps at the surface of the top layer are one of the most important sources of the 2DEG in the channel. However, this only applies for a specific top layer thickness. In a relatively thin top layer, the surface trap state is below the Fermi level. However, as the top layer thickness increases, the energy of the surface trap state approaches the Fermi energy until it coincides with it. The thickness of the top layer corresponding to such situation is defined as "critical". At this point, electrons filling the surface trap state are pulled to the channel by the strong polarisation-induced electric field found in the top layer to form the 2DEG instantly.

If the surface trap states are completely depleted, further increase in the top layer thickness will not increase the 2DEG density. Actually, if the 2DEG channel layer fails to stretch the top layer, the later will simply relax. Upon relaxation of the top layer, many crystal defects are created at the interface between the top layer and the layer right underneath it, and the piezoelectric polarisation instantly disappears causing deterioration in the 2DEG density.

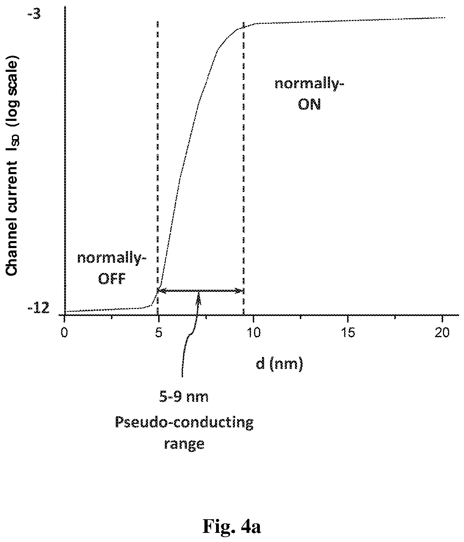

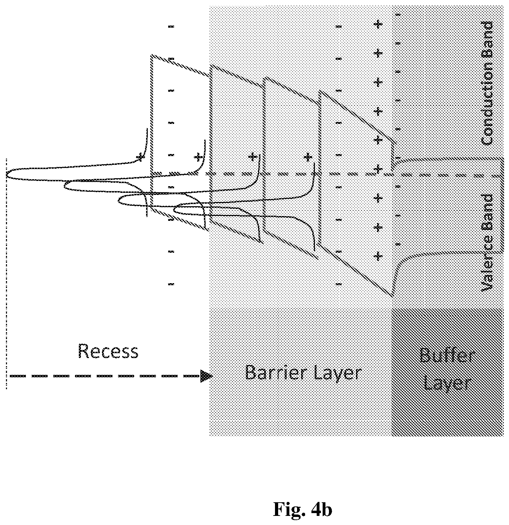

In order to illustrate the above phenomenon of the pseudo-conducting current, reference is now made to the following figures. **Fig. 4a** shows the dependence of the source-drain current (a charge carrier density) on the recessed AlGaN layer thickness. As seen from the plot, transistors that have a thickness of the top layer larger than 9 nm form normally-on 2DEG channels. In such transistors, due to the inherent polarisation effects present in the III-V materials, a thin sheet of charges is induced at the top and bottom of the interfaces of the top layer. As a result, a high electric field is induced in the top layer, and surface donor states at the top interface start donating electrons to form the 2DEG channel at the proximity of the hetero-junction interface without the application of a gate bias. These transistors therefore constitute normally-on devices. On the other hand, the transistors that have a thickness of the top layer lower than about 5 nm act constitute normally-off devices. Energy equilibrium between the donor surface trap states and AlGaN tunnel barrier leads to the 2DEG formation (charge neutrality combined with the lowest energy level) at the conduction band discontinuity. As explained above, decrease in the thickness of the AlGaN layer results in increase of the energy barrier. As a result, the ionisable donor-like surface trap states, which are responsible for electron tunnelling from the surface to 2DEG, drift bellow the Fermi level, thereby minimizing the electron supply to the 2DEG channel. This theoretical situation is further illustrated in **Fig. 4b****.** Therefore, the recess of the AlGaN layer from 9 nm to 5 nm leads to huge drop in conductivity of the two-dimensional electron gas for six orders of magnitude.

In view of the above, it is clear that the mechanism of the 2DEG depletion based on recessing the top layer is strongly dependent on the donor-like surface trap states (or total surface charge). As the thickness of the top layer decreases, less additional external charge is needed to apply to the top layer surface in order to deplete the 2DEG channel. There is a critical (smallest) barrier thickness, when the 2DEG channel is mostly depleted but still highly conductive due to a combination of the energy barrier and the donor surface trap states energy. At this critical thickness, even the smallest energy shift at the surface via any external influence, for example an acoustic wave propagating along the surface, leads immediately to the very strong 2DEG depletion. As a result, the surface of the top layer at this critical thickness is extremely sensitive to any smallest change in the electrical field of the surroundings. Thus, the recess of the top layer from 9 nm down to 5 nm significantly reduced the 2DEG density, brought the sensor to the "near threshold" operation and resulted in highly increased surface charge sensitivity. The specific 5-9 nm thickness of the top layer is actually responsible for the pseudo-conducting behaviour of the 2DEG channel and gives the sensor an incredible sensitivity.

The top layer is recessed to this specific thickness after subjecting to short plasma activation by an ultra-low damage reactive-ion etching technique using inductively-coupled plasma (ICP) with a narrow plasma-ion energy distribution. Such short plasma treatment allows much lower roughness of the surface, which is a function of the semiconductor vertical damage depth during the plasma etching process. Such low surface roughness (about 0.2 nm and less) can be achieved only via this ICP-RIE ultra low damage etching process with a narrow plasma-ion energy distribution, and this inherently results in a very low vertical damage depth to the top layer, which allows the minimal surface scattering and minimal surface states-2DEG channel interaction with the maximum signal-to-noise ratio of the sensor. Thus, the depth effect of the vertical sub-nanometre damage to the top recessed layer, due to an ultra-low damage ICP-RIE etching process with a very narrow plasma-ion energy distribution, is the only way to optimally achieve the required sub-nanometre roughness of the semiconductor surface. This inherently results in an adjustable pseudo-conductive working point with the highest charge sensitivity ever possible. This depth effect is always inherent to the sub-nanometre roughness of the semiconductor surface, which was measured using AFM (atomic force microscope).

Thus, in addition to the recessed top layer thickness, roughness of the top layer surface is another very important parameter that has not been previously disclosed. It has been surprisingly found that the roughness of the top layer surface (in the open gate sensitive area) bellow 0.2 nm prevents scattering of the donor-like surface trap states. Thus, combination of these two features: 5-9 nm thickness of the top layer in the open gate area and strongly reduced roughness of its surface (bellow 0.2 nm) make the sensor incredibly sensitive.

In a certain aspect, the method for manufacturing of the PC-HEMTs of the present invention comprises the following steps:
Step 1: Plasma-enhanced atomic layer deposition (ALD) of alumina (Al₂O₃) on a pre-aligned masked Si-GaN/AlGaN wafer with nitrogen-plasma de-trapping for the thickness of the Al₂O₃ layer being 3-10 nm. The Al₂O₃ layer thickness was measured with an X-ray reflectometer.
Step 2: Plasma-enhanced atomic layer deposition (ALD) pattering of the wafer coated with the thin Al₂O₃ layer in Step 1, with hydrogen fluoride (HF) or using the aforementioned reactive-ion etching (RIE) technique.
Step 3: Optionally creating the source and drain ohmic contacts (in case ohmic contacts are required) on the coated wafer obtained in Step 2 from metal stacks, for example Ti/Al/Mo/Au, Ti/Al/Ni/Au, Ti/Au and Ti/W, having 15-50 nm thickness, using spin-coating technique or e-beam physical vapour deposition (VPD) of the stack metals. The deposition rates using the e-VPD technique were determined for the ohmic-stack metals using the Dektak Profilometer with dummy lift-off samples.
Step 4: Two-dimensional electron gas (2DEG) channel-pattering of the wafer obtained in Step 3 with argon- or nitrogen-ion implantation.
Step 5: Plasma-enhanced chemical vapour deposition (CVD) of the ONO stack over the wafer obtained in Step 4. This is the stress-free technique to deposit the layer of the SiO-SiN-SiO stack having an exemplary thickness of about 200-300 nm and structured by the ICP-RIE dry etching, which is the CF4-based etching method. In this step, the pseudo-conducting channel areas and ohmic electrical contact pads of the transistor become available.
Step 6: Optional lift-off deposition of an Au or Ti/W-CMOS-gate electrode (in case a gate electrode is to be deposited on the top layer of the heterojunction structure for an integrated MMIC-HEMT-based amplifier manufacturing).
Step 7: Optional plasma-enhanced ALD pattering with RIE or HF above sensing area (in case the plasma-enhanced ALD layer deposited in Step 1 is removed separately to ONO stack).
Step 8: Atomic layer etching (ALE) of the wafer obtained in Steps 5-7. This sophisticated technique carried out in the clean manufacturing cluster of the applicant is the only technique allowing the removal of individual atomic layers (the top atomic layers of the wafer). ALE is a way better-controlled technique than RIE, though it has not been commercially used until now because very sophisticated gas handling is required, and removal rates of one atomic layer per second are the real state of the art. This step is the step of creating the pseudo-conducting working point of the transistor, because ALE allows achieving the specific thickness of 5-9 nm thickness of the top layer in the open gate area with the extremely low surface roughness of the top layer below 0.2 nm.
Step 9: Optional plasma-enhanced CVD or ALD of the dielectric layer used for device passivation and in some gas sensors.
Step 10: Optional deep reactive-ion etching (DRIE or Bosch process) of the Si-substrate under sensing areas (in case the substrate is on the free-standing membranes - used, for example, in RF-HEMTs, FBAR and SAW sensors).

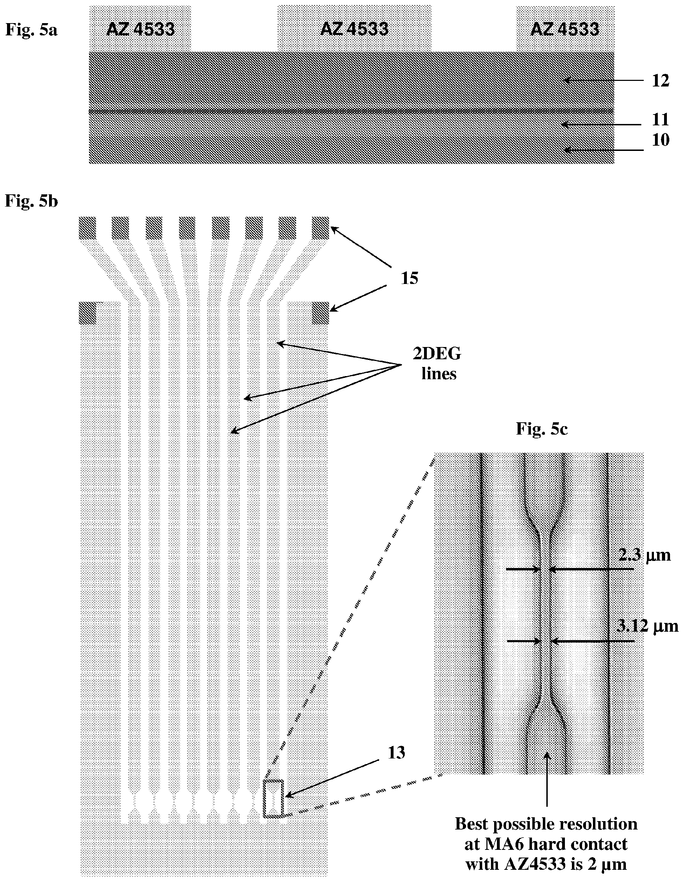

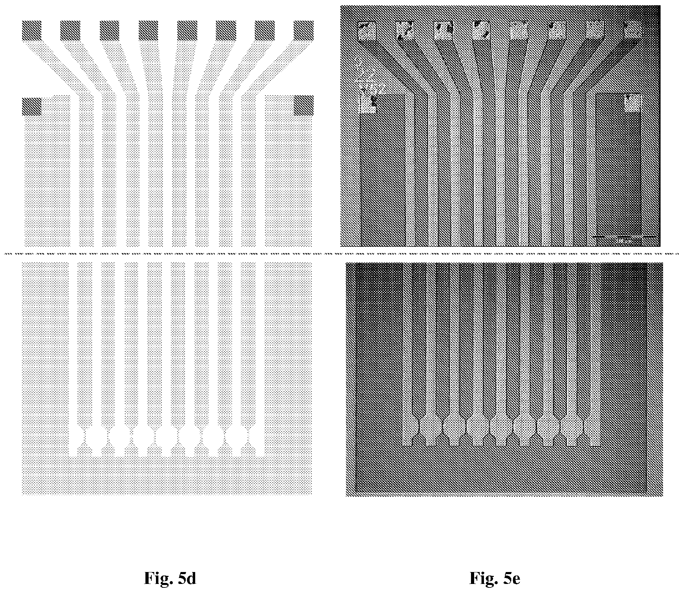

Reference is now made to **Figs. 5a-5c** showing the sensor, which is obtained in Step 4 of the 2DEG-channel pattering. The lithography of the sensor was performed with AZ 4533, which is a positive thick resist having optimised adhesion for common wet etching. The lithographic resist film thickness obtained at 7000-rpm spin speed and at 100° C for 1 min was 3 µm. Thus, as seen in the lithographic image of **Fig. 5c****,** the formed 2DEG channel (13) is approximately 2-3 µm wide. The overall exposure time was 9 sec, followed by 5-min development in MIF726 developer.

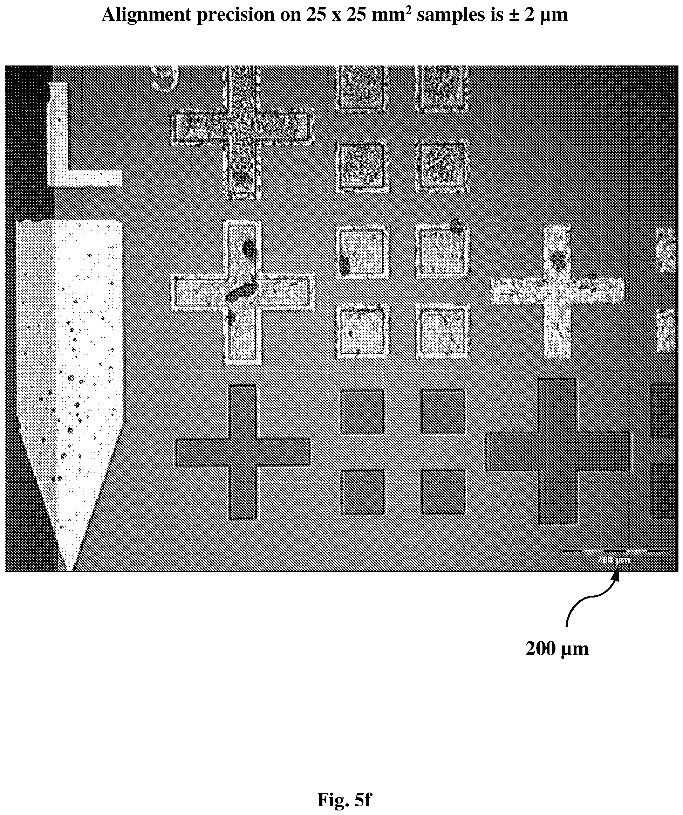

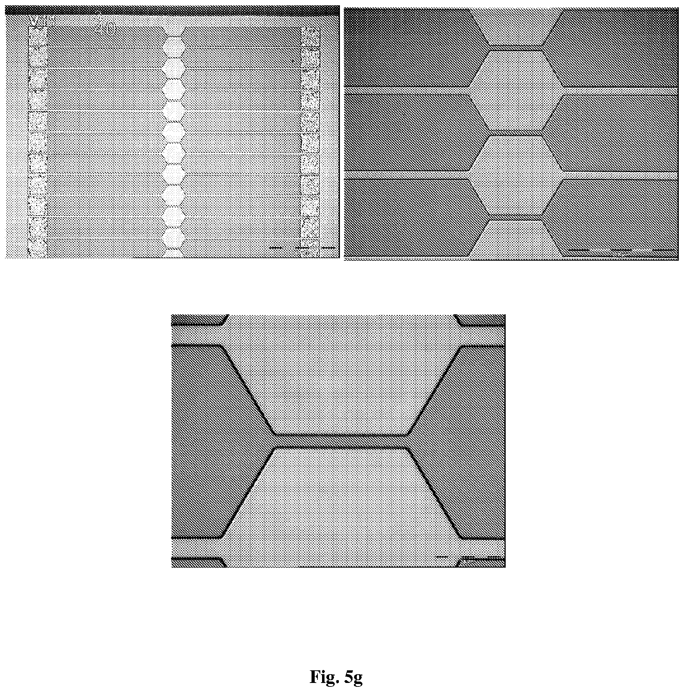

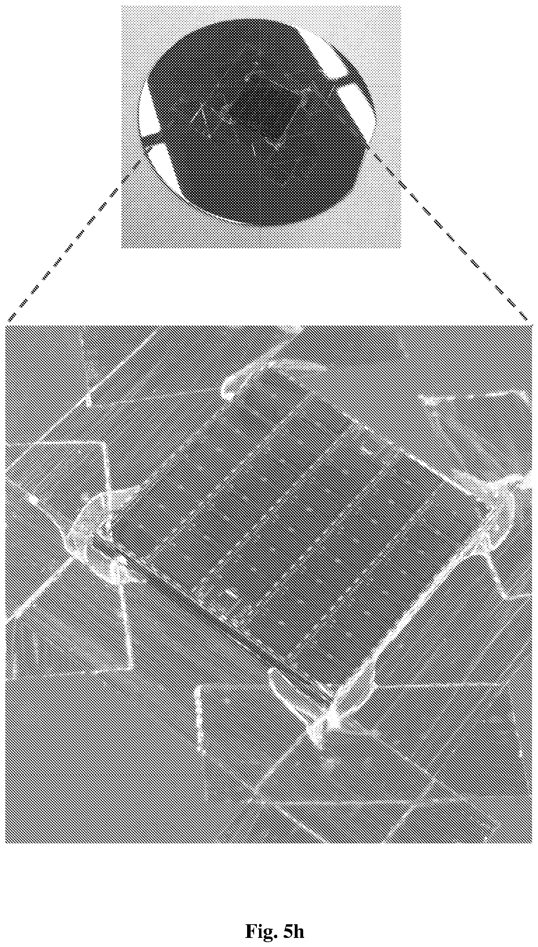

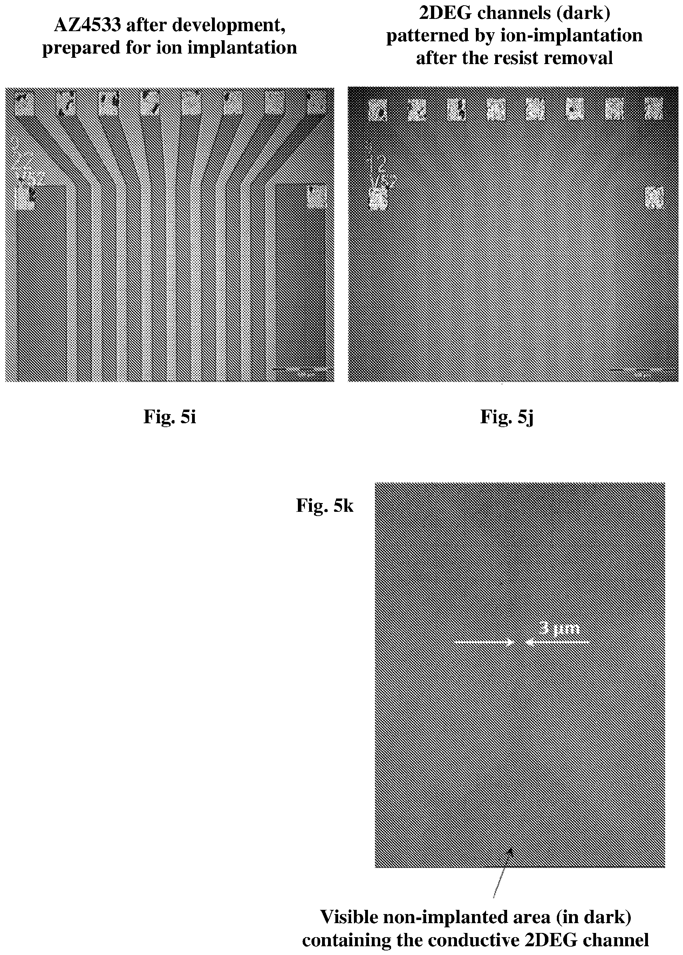

**Fig. 5d-5e** show the mask and corresponding lithographic image, respectively, of the sensor layout of the present invention. **Fig. 5f** demonstrates the high alignment precision of ± 2-µm on 25 x 25 mm² samples in the lithography of the sensor layout of the present invention. **Fig. 5g** shows the lithographic images of the multichannel samples. **Fig. 5h** shows the fixed sensor chip sample on the Si-GaN/AlGaN wafer, which contains approximately 30-32 sensors with 4-8 channels on each sample and prepared for ion implantation. **Fig. 5i** shows the obtained lithographic image of the present sensor layout with the AZ4533 resist after development, prepared for ion implantation. **Fig. 5j** shows the 2DEG channels (dark) patterned by ion-implantation after the resist removal. The argon-ion implantation was conducted with 20 keV and 30 keV energies and with an exemplary dose of 2.5e¹³/cm² and a 7° tilt angle. AZ4533 was removed with oxygen plasma at 220 W for 10 min. **Fig. 5k** shows the visible non-implanted area containing the conductive 2DEG channel.

The atomic layer etching (ALE) performed in Step 8 of the manufacturing process is the most important stage in the process. As mentioned above, it allows the controlled recess of a top layer, removing a single atomic layer-by-layer, where the etch thickness is in the order of magnitude of a single atomic monolayer. As explained above, such ultra-low damage to the top layer of the heterogeneous structure, when the actual surface roughness is controlled by a single atomic monolayer, allows to achieve the sub-nanometre roughness (about 0.2 nm and less) of the top layer when its thickness is only few nanometres (5-9 nm).

The ALE process sequence consists of repeated cycling of process conditions. The total amount of material removed is determined by the number of repeated cycles. Each cycle is typically comprised of four steps: adsorption, first purge, desorption and second purge. During the adsorption step of the cycle, reactive species are generated in the reactor (for example, upon plasma excitation), adsorbed by, and react with material on the wafer. Due to the self-limiting process, and with the proper choice of reactants and process conditions, reaction takes place with only a thin layer of material, and the reaction by-products are formed. This step is followed by purging of the reactor to remove all traces of the reactant. Then the by-product desorption takes place due to bombardment of the wafer surface by noble gas ions with a tightly controlled energy. Again, by-products are purged from the reactor, and the wafer is ready for the last two (optional) steps of the manufacturing process.

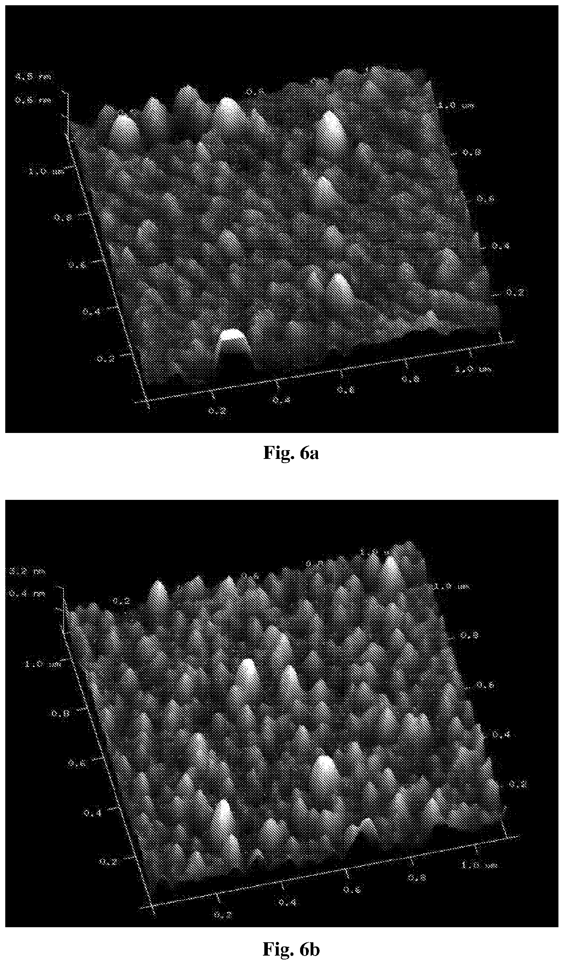

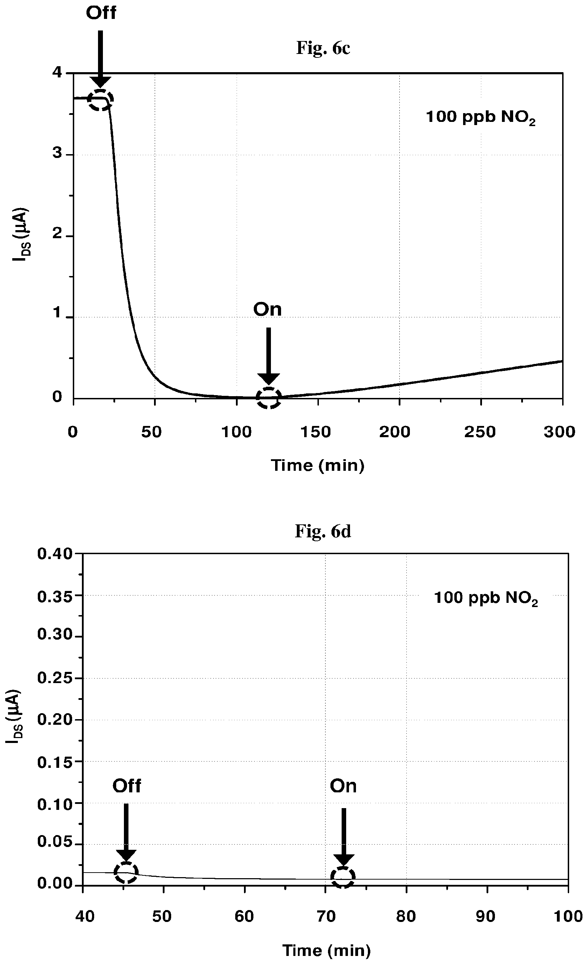

Reference is now made to **Fig. 6a** showing the AFM image of the top recessed layer surface of the PC-HEMT produced by the manufacturing process of the present invention. The measured RMS value of the surface roughness is 0.674 nm in this case. **Fig. 6b** shows the AFM surface image of the top recessed layer of the HEMT made by a conventional manufacturing process. In this conventional process, the HEMT initially had a top ultrathin-grown AlGaN layer of the 6-7 nm thickness. This layer was recessed with inductively-coupled plasma (ICP) for 60 sec using a conventional reactive-ion etching (RIE) technique. The measured RMS value of the surface roughness is 1.211 nm in this case. **Figs. 6c** show the time-dependent plot of the drain-source electric current I_{DS} of the nitrogen oxide sensor measuring 100 ppb of the NO₂ gas in 80%-humid air, where the sensor incorporates the PC-HEMT made by the manufacturing process of the present invention. **Figs. 6d** show the time-dependent plot of the I_{DS} of the nitrogen oxide sensor measuring 100 ppb of the NO₂ gas in 80%-humid air, where the sensor incorporates and based on the HEMT made by the conventional manufacturing process. It is clear from these comparative examples that the manufacturing process of the present invention based on the ultra-low damaging RIE with a narrow plasma-ion energy distribution leads to much lower roughness of the semiconductor surface, which in turn leads to incredibly high sensitivity of the sensor.

In a further aspect, the hetero-junction structure may be a three-layer structure consisting of two GaN layers and one AlGaN layer squeezed between said buffer layers like in a sandwich, wherein the top layer is a buffer layer. This may lead to formation of the two-dimensional hole gas (2DHG) in the top GaN layer above the AlGaN layer which results in reversing polarity of the transistor compared to the two-layer structure discussed above.

In general, polarity of III-V nitride semiconductor materials strongly affects the performance of the transistors based on these semiconductors. The quality of the wurtzite GaN materials can be varied by their polarity, because both the incorporation of impurities and the formation of defects are related to the growth mechanism, which in turn depends on surface polarity. The occurrence of the 2DEG/2DHG and the optical properties of the hetero-junction structures of nitride-based materials are influenced by the internal field effects caused by spontaneous and piezo-electric polarizations. Devices in all of the III-V nitride materials are fabricated on polar {0001} surfaces. Consequently, their characteristics depend on whether the GaN layers exhibit Ga-face positive polarity or N-face negative polarity. In other words, as a result of the wurtzite GaN materials polarity, any GaN layer has two surfaces with different polarities, a Ga-polar surface and an N-polar surface. A Ga-polar surface is defined herein as a surface terminating on a layer of Ga atoms, each of which has one unoccupied bond normal to the surface. Each surface Ga atom is bonded to three N atoms in the direction away from the surface. In contrast, an N-polar surface is defined as a surface terminating on a layer of N atoms, each of which has one unoccupied bond normal to the surface. Each surface N atom is also bonded to three Ga atoms in the direction away from the surface. Thus, the N-face polarity structures have the reverse polarity to the Ga-face polarity structures.

As described above for the two-layer heterojunction structure, the barrier layer is always placed on top of the buffer layer. The layer which is therefore recessed in the two-layer heterojunction structure is the barrier layer, specifically the AlGaN layer. As a result, since the 2DEG is used as the conducting channel and this conducting channel is located slightly below the barrier layer (in a thicker region of the GaN buffer layer), the hetero-junction structure is grown along the {0001}-direction or, in other words, with the Ga-face polarity. However, as explained above, the physical mechanism that leads to the formation of the 2DEG is a polarisation discontinuity at the AlGaN/GaN interface, reflected by the formation of the polarisation-induced fixed interface charges that attract free carriers to form a two-dimensional carrier gas. It is a positive polarisation charge at the AlGaN/GaN interface that attracts electrons to form 2DEG in the GaN layer slightly below this interface.

As noted above, polarity of the interface charges depends on the crystal lattice orientation of the hetero-junction structure, i.e. Ga-face versus N-face polarity, and the position of the respective AlGaN/GaN interface in the hetero-junction structure (above or below the interface). Therefore, different types of the accumulated carriers can be present in the hetero-junction structure of the embodiments.

In case of the three-layer hetero-junction structure, there are four possible configurations:

### Ga-face polarity

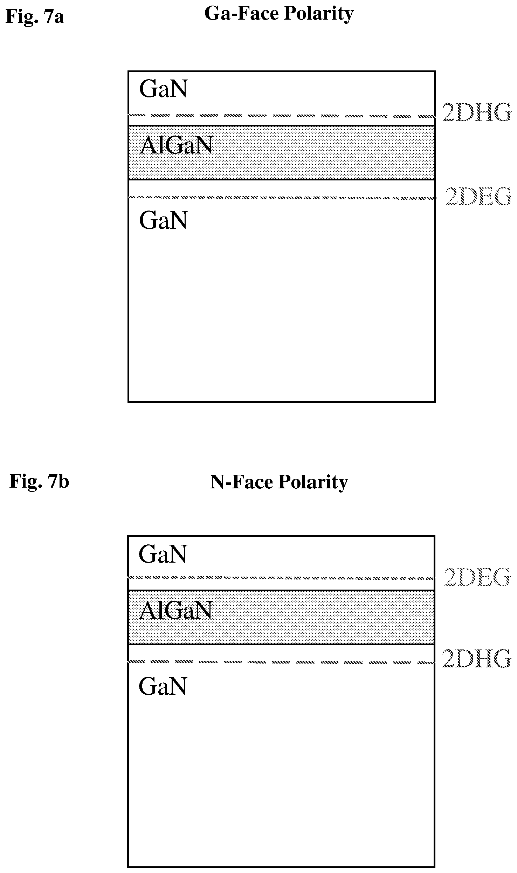

1) The Ga-face polarity is characterised by the 2DEG formation in the GaN layer below the AlGaN barrier layer. This is actually the same two-layer configuration as described above, but with addition of the top GaN layer. In this configuration, the AlGaN barrier layer and two GaN layers must be nominally undoped or n-type doped.
2) In another Ga-face configuration shown in **Fig. 7a****,** in order to form the conducting channel comprising a two-dimensional hole gas (2DHG) in the top GaN layer above the AlGaN barrier layer in the configuration, the AlGaN barrier layer should be p-type doped (for example, with Mg or Be as an acceptor) and the GaN buffer layer should be also p-type doped with Mg, Be or intrinsic.

### N-face polarity

3) The N-face polarity is characterised by the 2DEG formation in the top GaN layer above the AlGaN barrier layer, as shown in **Fig. 7b****.** In this case, the AlGaN barrier layer and two GaN buffer layers must be nominally undoped or n-type doped.
4) The last configuration assumes that the 2DHG conducting channel is formed in the buffer GaN layer below the AlGaN barrier layer. The top GaN layer may be present (three-layer structure) or not (two-layer structure) in this case. The AlGaN barrier layer must be p-type doped (for example, with Mg or Be as an acceptor) and the bottom GaN layer should be also p-type doped with Mg, Be or intrinsic.

Thus, there are four hetero-junction three-layer structures implemented in the transistor of the embodiments, based on the above configurations:
A. **Ga-Face** GaN/AlGaN/GaN heterostructure with the 2DEG formed in the GaN buffer layer below the AlGaN barrier layer. In this case, the top GaN layer may be omitted to obtain the two-layer structure. For the three-layer structure, the top GaN layer must be recessed to 1-9 nm thickness in the open gate area or grown with this low thickness, with the roughness below 0.2 nm, and the thickness of the AlGaN barrier can be adjusted properly during growth
B. **Ga-Face** GaN/AlGaN/GaN heterostructure with the 2DHG conducting channel formed in the top GaN layer above the AlGaN barrier layer. The top GaN layer must be recessed to 5-9 nm thickness in the open gate area with the roughness below 0.2 nm, and the thickness of the AlGaN barrier layer can be adjusted properly. P-type doping concentrations of the GaN layer and AlGaN barrier have to be adjusted; the 2DHG has to be contacted (in the ideal case by ohmic contacts).
C. **N-Face** GaN/AlGaN/GaN heterostructure with the 2DEG in the top GaN layer above the AlGaN barrier layer. The top GaN layer must be recessed to 5-9 nm thickness in the open gate area with the roughness below 0.2 nm. Thickness of the AlGaN barrier can be adjusted during growth. N-type doping levels of the GaN buffer layer and the AlGaN barrier layer must be adjusted; the 2DEG has to be contacted (in the ideal case by ohmic contacts).
D. **N-Face** GaN/AlGaN/GaN heterostructure with the 2DHG in the GaN buffer layer below the AlGaN barrier layer. In this case, the top GaN layer may be omitted to obtain the two-layer structure. In both, the two-layer and three-layer configurations, the top GaN layer must be recessed to 1-9 nm thickness in the open gate area with the roughness below 0.2 nm, and the thickness of the AlGaN barrier can be adjusted properly.

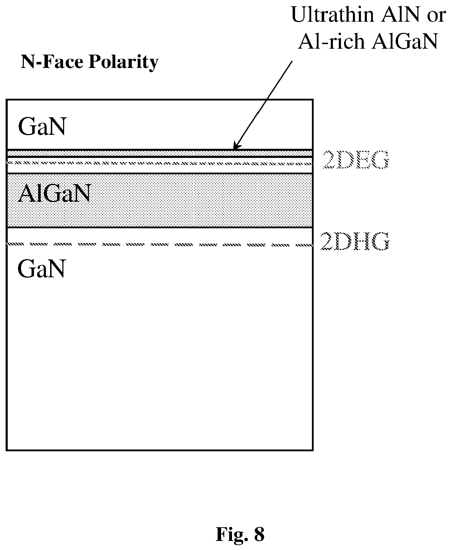

In all the above structures, the deposition of a dielectric layer on top might be beneficial or even necessary to obtain a better confinement (as in case of the N-face structures). As shown in **Fig. 8****,** for the above "C" structure, it may be even more beneficial to include an ultrathin (about 1 nm) A1N or AlGaN barrier layer with high Al-content on top of the 2DEG channel to improve the confinement.

The preferable structures of the embodiments are structures "B" and "C". In the structure "B", the 2DHG conducting channel formed in the top GaN layer, which has a higher chemical stability (particularly towards surface oxidation) than the AlGaN layer. Concerning the structure "C", the 2DEG conducting channel might be closer to the surface. Therefore, the electron mobility might be lower than in the 2DEG structure with the Ga-face polarity. In general, the polarity of the heterostructure can be adjusted by the choice of the substrate (e.g. C-face SiC) or by the growth conditions.

Based on the above, one of the aspects of the present application is an open-gate pseudo-conductive high-electron mobility transistor (PC-HEMT) for amplifying signals produced by a body of a user in a sub-THz radiation range, comprising:
(1) a multilayer hetero-junction structure made of gallium nitride (GaN) and aluminium gallium nitride (AlGaN) single-crystalline or polycrystalline semi-conductor materials, deposited on a substrate layer, and ***characterised in that:***
   (a) said structure comprises (i) one top GaN layer recessed in an open gate area of the transistor to the thickness of 5-9 nm and having the surface roughness of 0.2 nm or less, (ii) one bottom GaN buffer layer, and (iii) one AlGaN barrier layer in between; said layers have Ga-face polarity, thus forming a two-dimensional hole gas (2DHG) conducting channel in the top GaN layer, close to the interface with said AlGaN barrier layer;
   (b) said structure comprises (i) one top GaN layer recessed in an open gate area of the transistor to the thickness of 5-9 nm and having the surface roughness of 0.2 nm or less, (ii) one bottom GaN buffer layer, and (iii) one AlGaN barrier layer in between; said layers have N-face polarity, thus forming a two-dimensional electron gas (2DEG) conducting channel in the top GaN layer, close to the interface with said AlGaN barrier layer; or
   (c) said structure comprises (i) one top AlGaN layer recessed in an open gate area of the transistor to the thickness of 5-9 nm and having the surface roughness of 0.2 nm or less, and (ii) one bottom GaN buffer layer; said layers have N-face polarity, thus forming a two-dimensional hole gas (2DHG) conducting channel in the GaN buffer layer, close to the interface with said AlGaN barrier layer;
(2) source and drain contacts connected to said 2DEG or 2DHG conducting channel and to electrical metallizations for connecting said transistor to an electric circuit; and
(3) a Vivaldi antenna electrode placed on a top (GaN or AlGaN) layer between said source and drain contact areas in an open gate area of the transistor and capable of detecting electrical signals in the sub-THz-frequency range of 200-800 GHz.

Another important feature of the sensor of the present application is that an electrical connection of the heterojunction structure to the 2DEG or 2DHG channel is realised via capacitive coupling to the electrical metallizations through a Schottky barrier contact. "Capacitive coupling" is defined as an energy transfer within the same electric circuit or between different electric circuits by means of displacement currents induced by existing electric fields between circuit/s nodes. In general, ohmic contacts are the contacts that follow Ohm's law, meaning that the current flowing through them is directly proportional to the voltage. Non-ohmic contacts however do not follow the same linear relationship of the Ohm's law. In other words, electric current passing through non-ohmic contacts is not linearly proportional to voltage. Instead, it gives a steep curve with an increasing gradient, since the resistance in that case increases as the electric current increases, resulting in increase of the voltage across non-ohmic contacts. This is because electrons carry more energy, and when they collide with atoms in the conducting channel, they transfer more energy creating new high-energy vibrational states, thereby increasing resistance and temperature.

When electrical metallizations are placed over single-crystalline or polycrystalline semiconductor material, the "Schottky contact" or "Schottky barrier contact" between the metal and the semiconductor occurs. Energy of this contact is covered by the Schottky-Mott rule predicting the energy barrier between a metal and a semiconductor to be proportional to the difference of the metal-vacuum work function and semiconductor-vacuum electron affinity. However, this is an ideal theoretical behaviour, while in reality most interfaces between a metal and a semiconductor follow this rule only to some degree. The boundary of a semiconductor crystal abrupt by a metal creates new electron states within its band gap. These new electron states induced by a metal and their occupation push the centre of the band gap to the Fermi level. This phenomenon of shifting the centre of the band gap to the Fermi level as a result of a metal-semiconductor contact is defined as "Fermi level pinning", which differs from one semiconductor to another. If the Fermi level is energetically far from the band edge, the Schottky contact would preferably be formed. However, if the Fermi level is close to the band edge, an ohmic contact would preferably be formed. The Schottky barrier contact is a rectifying non-ohmic contact, which in reality is almost independent of the semi-conductor or metal work functions.

Thus, a non-ohmic contact allows electric current to flow only in one direction with a non-linear current-voltage curve that looks like that of a diode. On the contrary, an ohmic contact allows electric current to flow in both directions roughly equally within normal device operation range, with an almost linear current-voltage relationship that comes close to that of a resistor (hence, "ohmic").

Since the source and drain contacts are non-ohmic (i.e. capacitively-coupled), the DC readout cannot be carried out. To electrically contact the 2DEG/2DHG channel underneath, about 5-20 nm bellow the metallizations, the AC-frequency regime must be used. In other words, the AC readout or impedance measurements of the electric current flowing through the 2DEG/2DHG-channel should be performed in this particular case. The capacitive coupling of the non-ohmic metal contacts with the 2DEG/2DHG channel becomes possible only if sufficiently high AC frequency, higher than 30 kHz, is applied to the metallizations. To sum up, the electrical metallizations, which are capacitively coupled to the 2DEG/2DHG channel utilise the known phenomenon of energy transfer by displacement currents. These displacement currents are induced by existing electrical fields between the electrical metallizations and the 2DEG/2DHG conducting channel operated in the AC frequency mode through the Schottky contact as explained above.

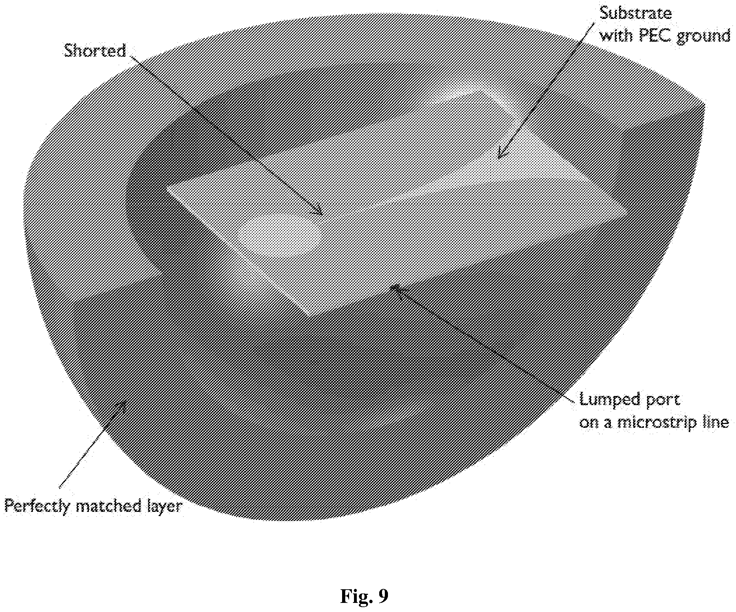

In one embodiment, a tapered slot antenna, also known as a Vivaldi antenna is placed in the open gate area of the transistor. The Vivaldi-antenna gate is capable of detecting various sub-THz-frequencies, i.e. 200-800 GHz. By applying additional plasmonic filters of any kind, the frequencies could be tuned precisely to a specific frequency of choice. In general, a Vivaldi antenna is a co-planar broadband-antenna, which is made from a dielectric plate metalized on both sides. **Fig. 9** shows the model the model of the Vivaldi antenna realised on a thin dielectric substrate. An exponential function is used for the taper profile. The entire domain is bounded by a perfectly matched layer. The objective of this model is to compute the far-field pattern and to compute the impedance of the structure. Good matching is observed over a wide frequency band. In this model of the Vivaldi antenna, the tapered slot is patterned with a perfect electric conductor (PEC) ground plane on the top of the Si dielectric substrate. One end of the slot is open to air and the other end is finished with a circular slot.

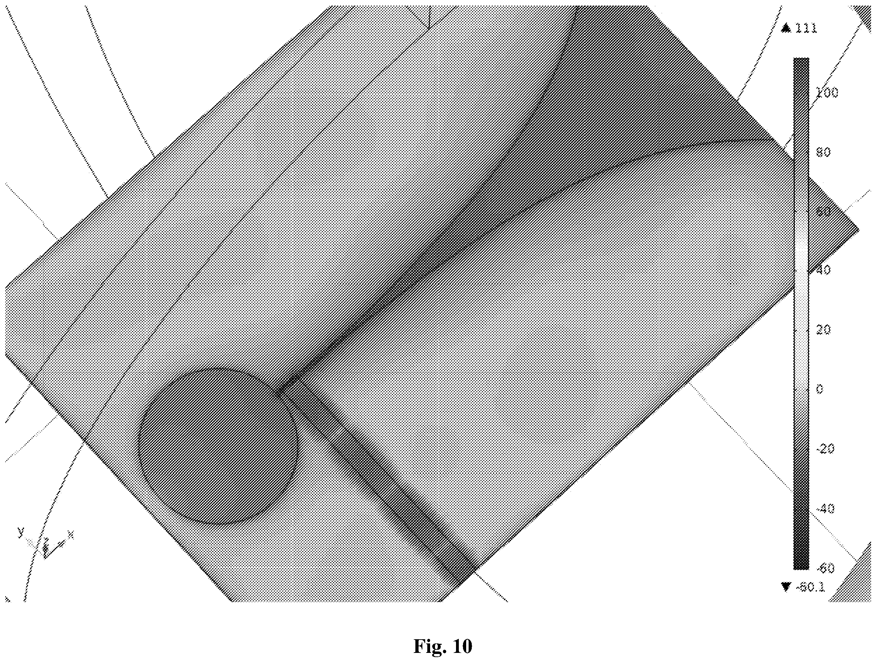

On the bottom of the substrate, the shortened 50 Ohm microstrip feed line is modelled as the PEC surfaces. The entire modeling domain is bounded by a perfectly matched layer (PML) chamber absorbing all radiated energy. To excite the antenna, a lumped port is used. **Fig. 10** shows the electric field distribution in the Vivaldi antenna plane at 480 GHz. The substrate size is 917x667x4 µm with total area about 0.6 mm². Substrate material is a high-dielectric constant, low conductive semiconductor material with σ > 1000 Obm·cm, such as for example, Si and GaAs. The following table provides the exemplary sub-THz Vivaldi antenna characteristics:

| **Parameter** | **Units** | **Value** | **Comments** |
|---|---|---|---|
| Substrate size | µm³ | 917x667x4 | Si, GaAs (high dielectric constant material) |
| Metal layer thickness | nm | 80 | Au |

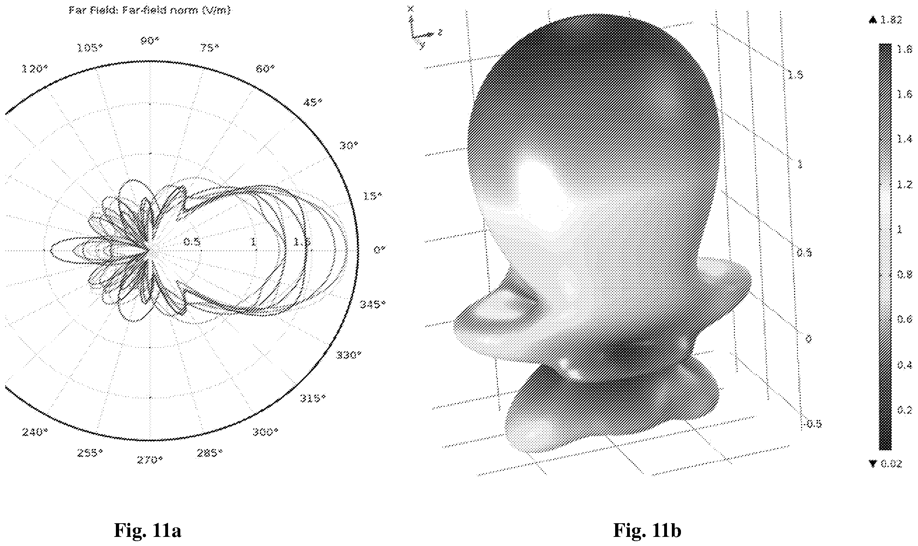

**Fig. 11** shows (a) the far-field directional radiation pattern of the Vivaldi antenna starting from 240 GHz till 780 GHz and (b) the corresponding 3D far-field pattern at 480 GHz, with the maximum in the X direction (axis along the substrate), which means this is a unidirectional antenna. As seen in **Figs. 10** and **11****,** the feeding line excites a circular space via a microstrip line, terminated with a sector-shaped area. From the circular resonant area the energy reaches an exponential pattern via a symmetrical slot line. In addition, the frequency response SWR of the Vivaldi antenna shows wide-band impedance matching better than 2:1 in most of the simulated frequency range. Printed circuit technology makes this type of antenna cost effective at sub-THz frequencies.

Advantages of the Vivaldi antenna is its broadband characteristics suitable for ultra-wideband signals in the sub-THz frequency domain, its easy manufacturing process using common methods for PCB production, and its easy impedance matching to the feeding line using microstrip line modelling methods. Also, the Vivaldi antenna has been chosen because it permits to integrate a long meander delay without having undesired effects.

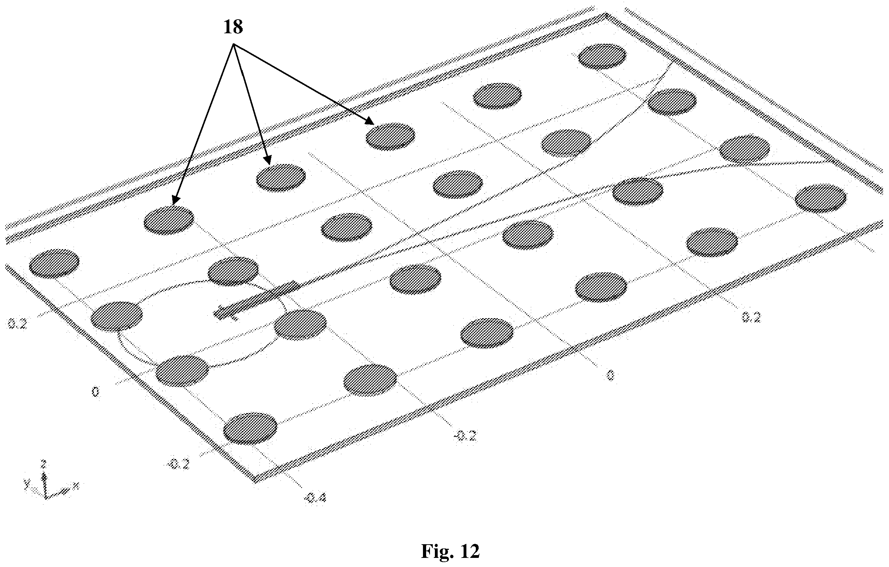

The Vivaldi antenna is a reciprocal device. It collects in a passive mode exactly the same frequencies that can be actively radiated. Due to its very broadband character, the Vivaldi antenna may receive signals outside the 240 GHz-780 GHz range. In order to limit the antenna sensitivity to the desired 0.3 THz-0.6 THz range, two-dimensional photonic crystals (18) may be superimposed on top of the antenna metal layer, as shown in **Fig. 12****.** The exemplary dimensions of such Vivaldi antenna and lattice constant of the photonic crystal are summarised in the following table:

| **Parameter** | **Units** | **Value** | **Comments** |
|---|---|---|---|
| Antenna substrate | µm | 4 | Si, GaAs (high dielectric constant material) |
| Device footprint area | µm² | 917x667 | |
| Metal layer thickness | nm | 80 | Au |
| Superimposed Si thickness | µm | 4 | Si or GaAs |
| Distance between the pillars | µm | 160 | Au or Al |
| Pillar diameter | µm | 60 | |
| Pillar height | µm | 4 | |

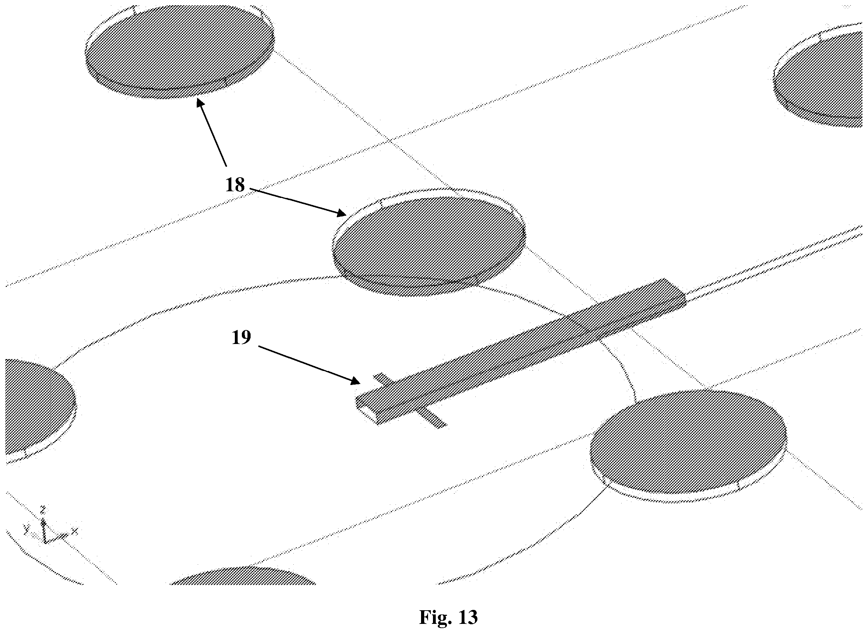

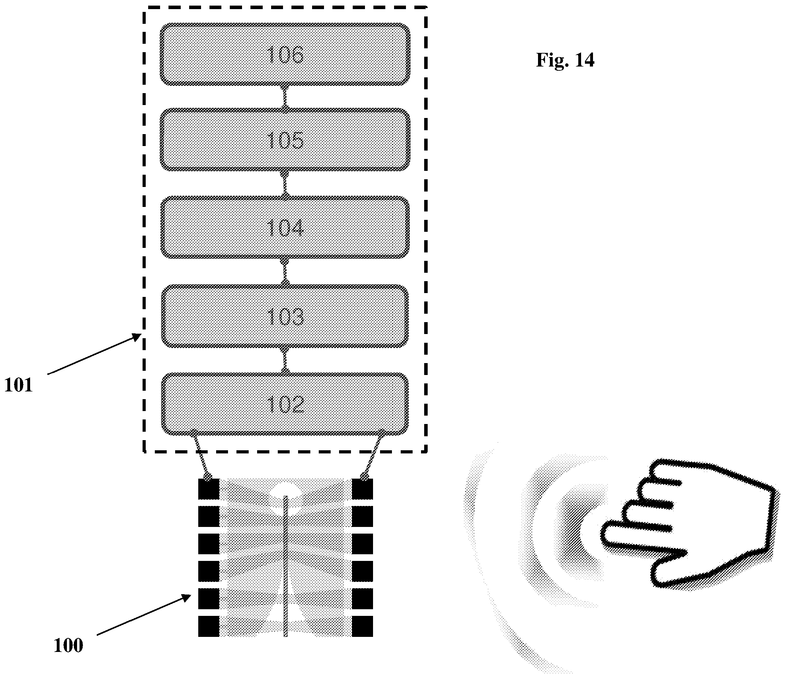

**Fig. 13** shows the position of the metal connector (19) on the Vivaldi antenna for coupling with the PC-HEMT of the embodiment. **Fig. 14** schematically shows a microelectronic sensor for biometric authentication, with a remote readout, comprising the following components (this is a single-transistor sub-THz solution):
(a) at least one PC-HEMT (100) of the present embodiments with an integrated Vivaldi antenna;
(b) an integrated circuit (101) for storing and processing a signal in a sub-THz frequency domain, and for modulating and demodulating a radio-frequency (RF) signals;
(c) an µ-pulse generator (102) for pulsed RF signal generation;
(d) an integrated DC-RF current amplifier or lock-in amplifier (103) connected to said µ-pulse generator (102) for amplification of the signal obtained from said µ-pulse generator;
(e) an analogue-to-digital converter (ADC) (104) with in-built digital input/output card connected to the amplifier (103) for converting the received analogue signal to a digital signal and outputting said digital signal to a microcontroller unit;
(f) the microcontroller unit (MCU) (105) for processing and converting the received digital signal into data readable in a user interface or external memory;
(g) a wireless connection module (106) for wireless connection of said sensor to said user interface or external memory.

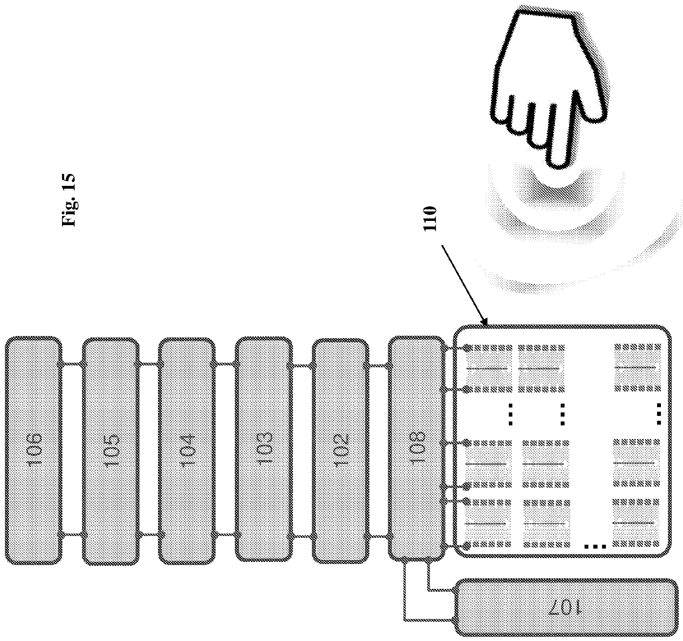

**Fig. 15** schematically shows a microelectronic sensor for biometric authentication, with a remote readout, comprising the following components (this is the DC/RF-based sub-THz antenna transistor-array solution for imaging):
(a) the array of the PC-HEMTs of the present embodiment (110), wherein each PC-HEMT in said array has an integrated Vivaldi antenna and connected to its dedicated electrical contact line;
(b) a row multiplexer (107) connected to said array for addressing a plurality of said transistors (PC-HEMTs) arranged in rows, selecting one of several analogue or digital input signals and forwarding the selected input into a single line;
(c) a column multiplexer (108) connected to said array for addressing a plurality of said transistors (PC-HEMTs) arranged in columns, selecting one of several analogue or digital input signals and forwarding the selected input into a single line;
(d) an integrated circuit for storing and processing said signals in a sub-THz frequency domain, and for modulating and demodulating a radio-frequency (RF) signals;
(e) an µ-pulse generator (102) for pulsed RF signal generation;
(f) an integrated DC-RF current amplifier or lock-in amplifier (103) connected to said µ-pulse generator (102) for amplification of the signal obtained from said µ-pulse generator;
(g) an analogue-to-digital converter (ADC) (104) with in-built digital input/output card connected to the amplifier (103) for converting the received analogue signal to a digital signal and outputting said digital signal to a microcontroller unit;
(h) the microcontroller unit (MCU) (105) for processing and converting the received digital signal into data readable in a user interface or external memory;
(i) a wireless connection module (106) for wireless connection of said microelectronic sensor to said user interface or external memory.

The ADC card (104) may be any suitable analogue-to-digital converter data logger card that can be purchased, for example, from National Instruments® or LabJack®. Optionally, the current amplifier (103) can be operated directly with current flowing via the 2DEG/2DHG channel into the amplifier with small input resistance of 1MΩ at gain higher than 10⁴ and only 1Ω at gains lower than 200. This setup may directly amplify the electric current modulation in the 2DEG channel originated from an external body charges.

In a specific embodiment, the wireless connection module (106) may be a short-range Bluetooth® or NFC providing wireless communication between the wearable device or gadget and a smartphone for up to 20 m. If this module is Wi-Fi, the connection can be established with a network for up to 200 nm, while GSM allows the worldwide communication to a cloud. The external memory may be a mobile device (such as a smartphone), desktop computer, server, remote storage, internet storage or telemedicine diagnostics cloud.

In some embodiments, the microelectronic sensor of the present application may be used for portable long-time-operation solution within remote cloud-based biometric authentication. The portable microelectronic sensor of the embodiments should have a very small power consumption saving the battery life for a prolong usage. In this case, the non-ohmic high-resistive contacts capacitively connecting the sensor to an electric circuit are preferable. The non-ohmic contacts actually limit an electric current flowing through the 2DEG/2DHG channel by having an electrical resistance 3-4 times higher than the resistance of the 2DEG/2DHG-channel, thereby reducing electrical power consumption without sacrificing sensitivity and functionality of the sensor. Thus, the use of non-ohmic contacts in some embodiments of the sensor of the present application is a hardware solution allowing to minimise the power consumption of the device. In another embodiment, the power consumption of the device can be minimised using a software algorithm managing the necessary recording time of the sensor and a battery saver mode, which limits the background data and switches the wireless connection only when it is needed.

In some embodiments, the microelectronic sensor may be integrated within a smartwatch, smartphone or in any other available personal gadget or wearable device, including but not limited to a bracelet, a ring or an earring, with or without any direct skin-contact to the sensor interface. It can be connected to the metallic chassis or to the capacitive sensitive display elements of the smartphone transducing an electrical charge to the sensor. The present sensor may replace the fingerprint sensor within the smartphone lock. The in-built present sensor is capable of sensing the signals and transmitting them either to a smartphone or directly to a biometric authentication cloud. The biometric authentication can be continuously carried out when the sensor is in a contact with a body, or activated on calling, or when the contact is established. The relevant biometric data recorded is then transmitted to a biometric authentication cloud and will be available for further processing. It may also be used in an automotive sector with car locks and bio-vital hemodynamic monitoring of the driver (sleepiness, cardio, stress etc.) In addition, the microelectronic sensor of the present application may be used in a biometric authentication chip of any security system, in a personal computer, laptop, credit card, any identification card or tag, in an automated teller machine, automatic gate opener, swing gate opener, flap barrier or turnstile gate.

In some embodiments, a method for biometric authentication of a user comprises the following steps:
(1) Contacting a single sensing point on the user's body with the microelectronic sensor of the embodiments, remotely positioning said sensor chip in a space against the user's body, or activating said sensor chip on calling or when the contact is established;
(2) Recording electrical signals received from the user's body with the microelectronic sensor in a form of a source-drain electric current of the microelectronic sensor over time (I_{DS} dynamics) and/or measuring S11-S12 parameters of the microelectronic sensor over time (S11-S12 dynamics);
(3) Transmitting the recorded signals from said microelectronic sensor to an external memory for further processing; and
(4) Converting the transmitted signals to digital signals, processing the digital signals in the external memory, and comparing said I_{DS} dynamics and/or S11-S12 dynamics with pre-calibrated biometric data of the user stored in the external memory, thereby biometrically authenticating the user.

In conclusion, what makes the sensor of the present embodiments particularly useful and unique is the combination of the PC-HEMT and Vivaldi antenna in one single transistor. The helical structure of human eccrine sweat ducts, together with the dielectric properties of the human skin, suggested that their electromagnetic (EM) properties would resemble those of an array of helical antennas. In order to examine the implications of this assumption, numerical simulations in the frequency range of 100-450 GHz, were conducted. In addition, an initial set of measurements was made, and the reflection spectrum measured from the skin of human subjects was compared to the simulation results. The simulation model consisted of a three layer skin model (dermis, epidermis, and stratum cornea) with rough boundaries between the layers and helical sweat ducts embedded into the epidermis. The spectral response obtained by the simulations coincided with the analytical prediction of antenna theory and supported the hypothesis that the sweat ducts can be regarded as helical antennas.

The results of the spectrum measurements from the human skin were found to be in a good agreement with the simulation results in the vicinity of the axial mode. The magnitude of this response was found to be dependent on conductivity of sweat in these frequencies, but the analysis of the phenomena and the frequencies related to the antenna-like modes were found to be independent of this parameter. Moreover, circular dichroism of the reflected electromagnetic field is a characteristic property of such helical antennas. The helical structure of the sweat ducts has the strongest effect on the reflected signal at frequencies above 200 GHz, where the wavelength and the dimensions of the ducts are comparable. In particular, the strongest spectral response (as calculated by the simulations and measured experimentally) was found to be around the predicted frequency (380 GHz) for the axial mode of the helical structure.

It was also experimentally proven, that besides the black body radiation between 1-20 THz, a human body actively irradiates sub-THz (0.1-1 THz) radiation from sweat ducts. Sweat ducts radiation may therefore accurately reflect the biometric pattern of an individual. Sweat ducts are emitting all the time independently of sweating process as they are always filled with a body electrolyte. Since every individual has his/her own sub-THz spectra and his/her own sweat-duct spatial skin-surface pattern, using this unique sub-THz-pattern may allow recording the very unique spectra and spatial duct pattern from each individual and then authenticating them with an unprecedented precision.

The Vivaldi antenna of the sensor chip of the embodiments is passively receiving these sub-THz signals from sweat ducts in approximately 200-800 GHz range. By applying additional plasmonic filter structures of any kind, the frequency may be precisely adjusted to a frequency of choice. The detection principle is based on the field effect current modulation in a DC-mode within the PC-HEMT 2DEG channel achieved by Vivaldi-shaped gate antenna. Once a user either touches the sensor or approached it at a relatively short distance (in a range of centimetres), the Vivaldi-shaped gate antenna may instantly and strongly concentrate the electric field strength of the sub-THz radiation in a very small area above the ultra charge sensitive 2DEG channel, thereby affecting its conductivity in the DC-mode by accumulation effect.

The S11-S12 parameters of the sensor may also be measured at RF frequencies of 1-60 GHz using beating effect from the sub-THz-radiation. By detecting the spatial sub-THz field distribution of the skin, an array containing 10-10,000 sub-THz-pixels may be used.

While certain features of the present application have been illustrated and described herein, many modifications, substitutions, changes, and equivalents will be apparent to those of ordinary skill in the art. The scope of the invention is defined by the claims.

## Claims

1. An open-gate pseudo-conductive high-electron mobility transistor for amplifying signals produced by a body of a user in a sub-THz radiation range, comprising:
(1) a multilayer hetero-junction structure made of gallium nitride (GaN) and aluminium gallium nitride (AlGaN) single-crystalline or polycrystalline semi-conductor materials, deposited on a substrate layer, wherein:
(a) said structure comprises (i) one top GaN layer recessed in an open gate area of the transistor to the thickness of 5-9 nm and having the surface roughness of 0.2 nm or less, (ii) one bottom GaN buffer layer, and (iii) one AlGaN barrier layer in between; said layers have Ga-face polarity, thus forming a two-dimensional hole gas (2DHG) conducting channel in the top GaN layer, close to the interface with said AlGaN barrier layer; or
(b) said structure comprises (i) one top GaN layer recessed in an open gate area of the transistor to the thickness of 5-9 nm and having the surface roughness of 0.2 nm or less, (ii) one bottom GaN buffer layer, and (iii) one AlGaN barrier layer in between; said layers have N-face polarity, thus forming a two-dimensional electron gas (2DEG) conducting channel in the top GaN layer, close to the interface with said AlGaN barrier layer; or
(c) said structure comprises (i) one top AlGaN layer recessed in an open gate area of the transistor to the thickness of 5-9 nm and having the surface roughness of 0.2 nm or less, and (ii) one bottom GaN buffer layer; said layers have N-face polarity, thus forming a two-dimensional hole gas (2DHG) conducting channel in the GaN buffer layer, close to the interface with said AlGaN barrier layer;
(2) source and drain contacts connected to said 2DEG or 2DHG conducting channel and to electrical metallizations for connecting said transistor to an electric circuit; and
(3) a Vivaldi antenna electrode placed on the top layer between said source and drain contact areas in an open gate area of the transistor and capable of detecting electrical signals in the sub-THz-frequency range of 200-800 GHz.

2. The transistor of claim 1, wherein the structure (b) further comprises an additional A1N or AlGaN layer having a high Al content and thickness of 1 nm or less, in the top GaN buffer layer above the 2DEG channel.

3. The transistor of claim 1, wherein said source and drain contacts are ohmic, or said electrical metallizations are capacitively-coupled to said 2DEG or 2DHG conducting channel for inducing displacement currents, thus resulting in said source and drain contacts being non-ohmic.

4. The transistor of claim 1, further comprising a dielectric layer deposited on top of said multilayer hetero-junction structure.

5. The transistor of claim 1, wherein the thickness of the top layer recessed in the open gate area of said transistor is 6-7 nm, or 6.2 nm to 6.4 nm.

6. The transistor of claims 1 or 5, wherein the surface roughness of the top layer recessed in the open gate area of said transistor is 0.1 nm or less, or 0.05 nm or less.

7. The transistor of claim 1, wherein said Vivaldi antenna electrode further comprises two-dimensional photonic crystals superimposed on top of its metal layer.

8. A microelectronic sensor for biometric authentication of a user, with a remote readout, comprising:
(a) at least one transistor (100) of any one of claims 1-7;
(b) an integrated circuit (101) for storing and processing a signal in a sub-THz frequency domain, and for modulating and demodulating a radio-frequency (RF) signals;
(c) an µ-pulse generator (102) for pulsed RF signal generation;
(d) an integrated DC-RF current amplifier or lock-in amplifier (103) connected to said µ-pulse generator (102) for amplification of the signal obtained from said µ-pulse generator;
(e) an analogue-to-digital converter (ADC) (104) with in-built digital input/output card connected to the amplifier (103) for converting the received analogue signal to a digital signal and outputting said digital signal to a microcontroller unit;
(f) the microcontroller unit (MCU) (105) for processing and converting the received digital signal into data readable in a user interface or external memory; and
(g) a wireless connection module (106) for wireless connection of said microelectronic sensor to said user interface or external memory.

9. A microelectronic sensor for biometric authentication of a user, with a remote readout, comprising:
(a) the array (110) of the transistors of any one of claims 1-7, wherein each transistor in said array has an integrated Vivaldi antenna and connected to its dedicated electrical contact line;
(b) a row multiplexer (107) connected to said array for addressing a plurality of said transistors arranged in rows, selecting one of several analogue or digital input signals and forwarding the selected input into a single line;
(c) a column multiplexer (108) connected to said array for addressing a plurality of said transistors arranged in columns, selecting one of several analogue or digital input signals and forwarding the selected input into a single line;
(d) an integrated circuit for storing and processing said signals in a sub-THz frequency domain, and for modulating and demodulating a radio-frequency (RF) signals;
(e) an µ-pulse generator (102) for pulsed RF signal generation;
(f) an integrated DC-RF current amplifier or lock-in amplifier (103) connected to said µ-pulse generator (102) for amplification of the signal obtained from said µ-pulse generator;
(g) an analogue-to-digital converter (ADC) (104) with in-built digital input/output card connected to the amplifier (103) for converting the received analogue signal to a digital signal and outputting said digital signal to a microcontroller unit;
(h) the microcontroller unit (MCU) (105) for processing and converting the received digital signal into data readable in a user interface or external memory; and
(i) a wireless connection module (106) for wireless connection of said microelectronic sensor to said user interface or external memory.

10. The microelectronic sensor of claim 8 or 9, wherein said external memory is a mobile device, desktop computer, server, remote storage, internet storage or biometric authentication cloud.

11. The microelectronic sensor of any one of claims 8-10, wherein said sensor is integrated within a smartwatch, smartphone or in any other available personal gadget or wearable device, with or without any direct skin-contact to the sensor interface.

12. The microelectronic sensor of claim 11, wherein said wearable device is a bracelet, a ring or an earring.

13. The microelectronic sensor of any one of claims 8-10, wherein said sensor is connected to a frame, or chassis, or capacitive sensitive display elements of a smartphone, smartwatch or personal electronic gadget, said smartphone, smartwatch or personal electronic gadget are capable of transducing an electrical charge to the sensor.

14. The microelectronic sensor of any one of claims 8-10, wherein said sensor is a fingerprint sensor within a smartphone lock or car lock, or said sensor is integrated in a biometric authentication module of any security system, personal computer, laptop, automatic gate opener, swing gate opener, flap barrier, turnstile gate, or automated teller machine, or said sensor is integrated in a biometric authentication chip of a credit card or any identification card or tag, or said sensor is suitable for use in bio-vital hemodynamic monitoring of a driver for the purpose of determining the sleepiness, tiredness, nervousness, cardio risk, stress and other conditions dangerous for driving a car.

15. A method for biometric authentication of a user comprising:
(1) Contacting a single sensing point on the user's body with the microelectronic sensor of any one of claims 8-14, or remotely positioning said sensor in a space against the user's body, and activating said sensor on calling or when the contact is established;
(2) Recording electrical signals received from the user's body with said microelectronic sensor in a form of a source-drain electric current of the sensor over time (I_{DS} dynamics) and/or measuring S₁₁-S₁₂ parameters of the sensor over time (S₁₁-S₁₂ dynamics);
(3) Transmitting the recorded signals from said sensor to an external memory for further processing; and
(4) Converting the transmitted signals to digital signals, processing the digital signals in the external memory, and comparing said I_{DS} dynamics and/or S₁₁-S₁₂ dynamics with pre-calibrated biometric data of the user stored in the external memory, thereby biometrically authenticating the user.

## Patentansprüche

1. Pseudo-Ieitfähiger Open-Gate-Transistor mit hoher Elektronenmobilität zum Verstärken von Signalen, die von einem Körper eines Benutzers in einem Sub-THz-Strahlungsbereich erzeugt werden, umfassend:
(1) eine mehrschichtige Heteroübergangsstruktur gefertigt aus einkristallinen oder polykristallinen Galliumnitrid- (GaN-) und Aluminiumgalliumnitrid- (AlGaN-) Halbleitermaterialien, die auf einer Substratschicht abgeschieden sind, wobei:
(a) die Struktur (i) eine obere GaN-Schicht, die in einem offenen Gate-Bereich des Transistors mit einer Stärke von 5-9 nm eingelassen ist und eine Oberflächenrauigkeit von 0,2 nm oder weniger aufweist, (ii) eine untere GaN-Pufferschicht und (iii) eine AlGaN-Barriereschicht dazwischen umfasst; wobei die Schichten eine Ga-seitige Polarität aufweisen, wodurch ein zweidimensionaler Lochgas- (2DHG-) Leitungskanal in der oberen GaN-Schicht nahe der Grenzfläche mit der AlGaN-Barriereschicht gebildet ist; oder
(b) die Struktur (i) eine obere GaN-Schicht, die in einem offenen Gate-Bereich des Transistors mit einer Stärke von 5-9 nm eingelassen ist und eine Oberflächenrauigkeit von 0,2 nm oder weniger aufweist, (ii) eine untere GaN-Pufferschicht und (iii) eine AlGaN-Barriereschicht dazwischen umfasst; wobei die Schichten eine N-seitige Polarität aufweisen, wodurch ein zweidimensionaler Elektrongas- (2DEG-) Leitungskanal in der oberen GaN-Schicht nahe der Grenzfläche mit der AlGaN-Barriereschicht gebildet ist; oder
(c) die Struktur (i) eine obere AlGaN-Schicht, die in einem offenen Gate-Bereich des Transistors mit einer Stärke von 5-9 nm eingelassen ist und eine Oberflächenrauigkeit von 0,2 nm oder weniger aufweist, und (ii) eine untere GaN-Pufferschicht umfasst; wobei die Schichten eine N-seitige Polarität aufweisen, wodurch ein zweidimensionaler Lochgas-(2DHG-) Leitungskanal in der GaN-Pufferschicht nahe der Grenzfläche mit der AlGaN-Barriereschicht gebildet ist;
(2) Source- und Drain-Kontakte, die mit dem 2DEG- oder 2DHG-Leitungskanal und mit elektrischen Metallisierungen verbunden sind, um den Transistor mit einer elektrischen Schaltung zu verbinden; und
(3) eine Vivaldi-Antennenelektrode, die auf der oberen Schicht zwischen den Source-und Drain-Kontaktbereichen in einem offenen Gate-Bereich des Transistors angeordnet ist und elektrische Signale in dem Sub-THz-Frequenzbereich von 200-800 GHz erfassen kann.

2. Transistor nach Anspruch 1, wobei die Struktur (b) ferner eine zusätzliche AlN-oder AlGaN-Schicht, die einen hohen Al-Gehalt und eine Stärke von 1 nm oder weniger aufweist, in der oberen GaN-Pufferschicht über dem 2DEG-Kanal umfasst.

3. Transistor nach Anspruch 1, wobei die Source- und Drain-Kontakte ohmsch sind, oder die elektrischen Metallisierungen kapazitiv mit dem 2DEG- oder 2DHG-Leitungskanal gekoppelt sind, um Verschiebungsströme zu induzieren, was darin resultiert, dass die Source- und Drain-Kontakte nicht ohmsch sind.

4. Transistor nach Anspruch 1, ferner umfassend eine dielektrische Schicht, die auf der mehrschichtigen Heteroübergangsstruktur abgeschieden ist.

5. Transistor nach Anspruch 1, wobei die Stärke der oberen Schicht, die in dem offenen Gate-Bereich des Transistors eingelassen ist, 6-7 Nm, oder 6,2 Nm bis 6,4 Nm ist.

6. Transistor nach Anspruch 1 oder 5, wobei die Oberflächenrauigkeit der oberen Schicht, die in dem offenen Gate-Bereich des Transistors eingelassen ist, 0,1 Nm oder weniger oder 0,05 Nm oder weniger ist.

7. Transistor nach Anspruch 1, wobei die Vivaldi-Antennenelektrode ferner zweidimensionale photonische Kristalle umfasst, die auf ihrer Metallschicht überlagert sind.

8. Mikroelektronischer Sensor für biometrische Authentifizierung eines Benutzers, mit einer Fernanzeige, umfassend:
(a) mindestens einen Transistor (100) nach einem der Ansprüche 1-7;
(b) eine integrierte Schaltung (101) zum Speichern und Verarbeiten eines Signals in einem Sub-THz-Frequenzbereich und zum Modulieren und Demodulieren eines Radiofrequenzsignals (RF-Signal);
(c) einen µ-Impuls-Generator (102) zur gepulsten RF-Signalerzeugung;
(d) einen integrierten DC-RF-Stromverstärker oder Lock-in-Verstärker (103), der mit dem µ-Impulsgenerator (102) verbunden ist, um das von dem µ-Impulsgenerator erlangte Signal zu verstärken;
(e) einen Analog-Digital-Wandler (ADC) (104) mit integrierter digitaler Eingangs-/Ausgangskarte, die mit dem Verstärker (103) verbunden ist, um das empfangene analoge Signal in ein digitales Signal umzuwandeln und das digitale Signal an eine Mikrocontrollereinheit auszugeben;
(f) die Mikrocontrollereinheit (MCU) (105) zum Verarbeiten und Umwandeln des empfangenen digitalen Signals in Daten, die in einer Benutzerschnittstelle oder einem externen Speicher lesbar sind; und
(g) ein drahtloses Verbindungsmodul (106) zur drahtlosen Verbindung des mikroelektronischen Sensors mit der Benutzerschnittstelle oder dem externen Speicher.

9. Mikroelektronischer Sensor für biometrische Authentifizierung eines Benutzers, mit einer Fernanzeige, umfassend:
(a) die Anordnung (110) der Transistoren nach einem der Ansprüche 1-7, wobei jeder Transistor in der Anordnung eine integrierte Vivaldi-Antenne aufweist und mit seiner dedizierten elektrischen Kontaktleitung verbunden ist;
(b) einen Zeilen-Multiplexer (107), der mit der Anordnung verbunden ist, um eine Vielzahl der in Zeilen angeordneten Transistoren zu adressieren, eines von mehreren analogen oder digitalen Eingangssignalen auszuwählen und den ausgewählten Eingang in eine einzelne Leitung einzugeben;
(c) einen Spalten-Multiplexer (108), der mit der Anordnung verbunden ist, um eine Vielzahl der in Spalten angeordneten Transistoren zu adressieren, eines von mehreren analogen oder digitalen Eingangssignalen auszuwählen und den ausgewählten Eingang in eine einzelne Leitung einzugeben;
(d) eine integrierte Schaltung zum Speichern und Verarbeiten der Signale in einem Sub-THz-Frequenzbereich und zum Modulieren und Demodulieren eines Radiofrequenzsignals (RF-Signal);
(e) einen µ-Impuls-Generator (102) zur gepulsten RF-Signalerzeugung;
(f) einen integrierten DC-RF-Stromverstärker oder Lock-in-Verstärker (103), der mit dem µ-Impulsgenerator (102) verbunden ist, um das von dem µ-Impulsgenerator erlangte Signal zu verstärken;
(g) einen Analog-Digital-Wandler (ADC) (104) mit integrierter digitaler Eingangs-/Ausgangskarte, die mit dem Verstärker (103) verbunden ist, um das empfangene analoge Signal in ein digitales Signal umzuwandeln und das digitale Signal an eine Mikrocontrollereinheit auszugeben;
(h) die Mikrocontrollereinheit (MCU) (105) zum Verarbeiten und Umwandeln des empfangenen digitalen Signals in Daten, die in einer Benutzerschnittstelle oder einem externen Speicher lesbar sind; und
(i) ein drahtloses Verbindungsmodul (106) zur drahtlosen Verbindung des mikroelektronischen Sensors mit der Benutzerschnittstelle oder dem externen Speicher.

10. Mikroelektronischer Sensor nach Anspruch 8 oder 9, wobei der externe Speicher eine mobile Vorrichtung, ein Desktop-Computer, ein Server, ein entfernter Speicher, ein Internetspeicher oder eine Cloud für biometrische Authentifizierung ist.

11. Mikroelektronischer Sensor nach einem der Ansprüche 8-10, wobei der Sensor in eine Smartwatch, ein Smartphone oder in ein anderes verfügbares persönliches Gadget oder eine tragbare Vorrichtung integriert ist, mit oder ohne direkten Hautkontakt zu der Sensorschnittstelle.

12. Mikroelektronischer Sensor nach Anspruch 11, wobei die tragbare Vorrichtung ein Armband, ein Ring oder ein Ohrring ist.

13. Mikroelektronischer Sensor nach einem der Ansprüche 8-10, wobei der Sensor mit einem Rahmen oder Gehäuse oder kapazitiv empfindlichen Anzeigeelementen eines Smartphones, einer Smartwatch oder eines persönlichen elektronischen Geräts verbunden ist, wobei das Smartphone, die Smartwatch oder das persönliche elektronische Gerät in der Lage sind, eine elektrische Ladung an den Sensor zu übertragen.

14. Mikroelektronischer Sensor nach einem der Ansprüche 8-10, wobei der Sensor ein Fingerabdrucksensor in einem Smartphone-Schloss oder Autoschloss ist, oder der Sensor in ein biometrisches Authentifizierungsmodul eines Sicherheitssystems, eines PCs, eines Laptops, eines automatischen Toröffners, eines Drehtoröffners, einer Klappschranke, eines Drehkreuzes oder eines Geldautomaten integriert ist, oder der Sensor in einen biometrischen Authentifizierungschip einer Kreditkarte oder einer Identifizierungskarte oder eines Identifizierungsanhängers integriert ist, oder der Sensor für eine Verwendung beim biovitalen hämodynamischen Überwachen eines Fahrers geeignet ist, um Schläfrigkeit, Müdigkeit, Nervosität, kardiales Risiko, Stress und andere für das Autofahren gefährliche Zustände zu bestimmen.

15. Verfahren für biometrische Authentifizierung eines Benutzers, umfassend:
(1) Inkontaktbringen eines einzelnen Erfassungspunkts am Körper des Benutzers mit dem mikroelektronischen Sensor nach einem der Ansprüche 8-14 oder Fernpositionierung des Sensors in einem Raum an den Körper des Benutzers und Aktivieren des Sensors bei Ruf oder wenn der Kontakt hergestellt ist;
(2) Aufzeichnen der von dem Körper des Benutzers empfangenen elektrischen Signale mit dem mikroelektronischen Sensor in Form eines elektrischen Source-Drain-Stroms des Sensors über die Zeit (ID_{S}-Dynamik) und/oder Messen von S₁₁-S₁₂-Parametern des Sensors über die Zeit (S₁₁-S₁₂-Dynamik);
(3) Übertragen der aufgezeichneten Signale von dem Sensor an einen externen Speicher zum weiteren Verarbeiten; und
(4) Umwandeln der übertragenen Signale in digitale Signale, Verarbeiten der digitalen Signale in dem externen Speicher und Vergleichen der I_{DS}-Dynamik und/oder S11-S12-Dynamik mit vorkalibrierten biometrischen Daten des Benutzers, die in dem externen Speicher gespeichert sind, wodurch der Benutzer biometrisch authentifiziert wird.

## Revendications

1. Transistor à haute mobilité électronique pseudo-conducteur à grille ouverte pour amplifier des signaux produit par un corps d'un utilisateur dans une plage de rayonnement sub-THz, comprenant :
(1) une structure d'hétérojonction multicouche constituée de matériaux semiconducteurs monocristallins ou polycristallins de nitrure de gallium (GaN) et de nitrure d'aluminium-gallium (AlGaN), déposée sur une couche de substrat, dans lequel :
(a) ladite structure comprend (i) une couche de GaN de dessus évidée dans une zone de grille ouverte du transistor à l'épaisseur de 5 à 9 nm et ayant la rugosité de surface de 0,2 nm ou moins, (ii) une couche tampon de GaN de dessous, et (iii) une couche barrière d'AlGaN entre elles ; lesdites couches ont une polarité de face Ga, formant ainsi un canal conducteur de gaz de trou bidimensionnel (2DHG) dans la couche de GaN de dessus, près de l'interface avec ladite couche barrière d'AlGaN ; ou
(b) ladite structure comprend (i) une couche de GaN de dessus évidée dans une zone de grille ouverte du transistor à l'épaisseur de 5 à 9 nm et ayant la rugosité de surface de 0,2 nm ou moins, (ii) une couche tampon de GaN de dessous, et (iii) une couche barrière d'AlGaN entre elles ; lesdites couches ont une polarité de face N, formant ainsi un canal conducteur de gaz d'électrons bidimensionnel (2DEG) dans la couche de GaN de dessus, près de l'interface avec ladite couche barrière d'AlGaN ; ou
(c) ladite structure comprend (i) une couche de d'AlGaN de dessus évidée dans une zone de grille ouverte du transistor à l'épaisseur de 5 à 9 nm et ayant la rugosité de surface de 0,2 nm ou moins, et (ii) une couche tampon de GaN de dessous ; lesdites couches ont une polarité de face N, formant ainsi un canal conducteur de gaz de trou bidimensionnel (2DHG) dans la couche tampon de GaN, près de l'interface avec ladite couche barrière d'AlGaN ;
(2) des contacts de source et de drain connectés audit canal conducteur 2DEG ou 2DHG et à des métallisations électriques pour connecter ledit transistor à un circuit électrique ; et
(3) une électrode d'antenne Vivaldi placée sur la couche de dessus entre les lesdites zones de contact de source et de drain dans une zone de grille ouverte du transistor et capable de détecter des signaux électriques dans la plage de fréquence sub-THz de 200 à 800 GHz.

2. Transistor selon la revendication 1, dans lequel la structure (b) comprend en outre une couche d'AIN ou d'AlGaN supplémentaire ayant une haute teneur en Al et une épaisseur de 1 nm ou moins, dans la couche tampon de GaN de dessus au-dessus du canal 2DEG.

3. Transistor selon la revendication 1, dans lequel lesdits contacts de source et de drain sont ohmiques, ou lesdites métallisations électriques sont couplées de manière capacitive audit canal conducteur 2DEG ou 2DHG pour induire des courants de déplacement, aboutissant ainsi au fait que lesdits contacts de source et de drain sont non ohmiques.

4. Transistor selon la revendication 1, comprenant en outre une couche diélectrique déposée sur le dessus de ladite structure d'hétérojonction multicouche.

5. Transistor selon la revendication 1, dans lequel l'épaisseur de la couche de dessus évidée dans la zone de grille ouverte dudit transistor est de 6 à 7 m, ou de 6,2 nm à 6,4 nm.

6. Transistor selon les revendications 1 ou 5, dans lequel la rugosité de surface de la couche de dessus évidée dans la zone de grille ouverte dudit transistor est de 0,1 nm ou moins, ou de 0,05 nm ou moins.

7. Transistor selon la revendication 1, dans lequel ladite électrode d'antenne Vivaldi comprend en outre des cristaux photoniques bidimensionnels superposés sur le dessus de sa couche métallique.

8. Capteur micro-électronique pour une authentification biométrique d'un utilisateur, avec une lecture à distance, comprenant :
(a) au moins un transistor (100) de l'une quelconque des revendications 1 à 7 ;
(b) un circuit intégré (101) pour stocker et traiter un signal dans un domaine de fréquence sub-THz, et pour moduler et démoduler des signaux radiofréquence (RF) ;
(c) un générateur d'µ-impulsions (102) pour une génération de signaux RF à impulsions ;
(d) un amplificateur de courant CC-RF intégré ou amplificateur synchrone (103) connecté audit générateur d'µ-impulsions (102) pour une amplification du signal obtenu depuis ledit générateur d'µ-impulsions ;
(e) un convertisseur analogique-numérique (CAN) (104) avec carte d'entrée/de sortie numérique incorporée connecté à l'amplificateur (103) pour convertir le signal analogique reçu en signal numérique et fournir en sortie ledit signal numérique à une unité de microcontrôleur ;
(f) l'unité de microcontrôleur (MCU) (105) pour traiter et convertir le signal numérique reçu en données lisibles dans une interface utilisateur ou mémoire externe ; et
(g) un module de connexion sans fil (106) pour une connexion sans fil dudit capteur micro-électronique à ladite interface utilisateur ou mémoire externe.

9. Capteur micro-électronique pour une authentification biométrique d'un utilisateur, avec une lecture à distance, comprenant :
(a) le réseau (110) des transistors (100) de l'une quelconque des revendications 1 à 7, dans lequel chaque transistor dans ledit réseau a une antenne Vivaldi intégrée et est connecté à sa ligne de contact électrique dédiée ;
(b) un multiplexeur de rangée (107) connecté audit réseau pour adresser une pluralité desdits transistors agencés en rangées, sélectionner l'un de plusieurs signaux d'entrée analogiques ou numériques et réacheminer l'entrée sélectionnée dans une seule ligne ;
(c) un multiplexeur de colonne (108) connecté audit réseau pour adresser une pluralité desdits transistors agencés en colonnes, sélectionner l'un de plusieurs signaux d'entrée analogiques ou numériques et réacheminer l'entrée sélectionnée dans une seule ligne ;
(d) un circuit intégré pour stocker et traiter lesdits signaux dans un domaine de fréquence sub-THz, et pour moduler et démoduler des signaux radiofréquence (RF) ;
(e) un générateur d'µ-impulsions (102) pour une génération de signaux RF à impulsions ;
(f) un amplificateur de courant CC-RF intégré ou amplificateur synchrone (103) connecté audit générateur d'µ-impulsions (102) pour une amplification du signal obtenu depuis ledit générateur d'µ-impulsions ;
(g) un convertisseur analogique-numérique (CAN) (104) avec carte d'entrée/de sortie numérique incorporée connecté à l'amplificateur (103) pour convertir le signal analogique reçu en signal numérique et fournir en sortie ledit signal numérique à une unité de microcontrôleur ;
(h) l'unité de microcontrôleur (MCU) (105) pour traiter et convertir le signal numérique reçu en données lisibles dans une interface utilisateur ou mémoire externe ; et
(i) un module de connexion sans fil (106) pour une connexion sans fil dudit capteur micro-électronique à ladite interface utilisateur ou mémoire externe.

10. Capteur micro-électronique selon la revendication 8 ou 9, dans lequel ladite mémoire externe est un dispositif mobile, un ordinateur de bureau, un serveur, un stockage distant, un stockage Internet ou un Cloud d'authentification biométrique.

11. Capteur micro-électronique selon l'une quelconque des revendications 8 à 10, dans lequel ledit capteur est intégré au sein d'une montre intelligente, d'un téléphone intelligent ou de tout autre gadget personnel ou dispositif portatif disponible, avec ou sans contact direct de la peau avec l'interface de capteur.

12. Capteur micro-électronique selon la revendication 11, dans lequel ledit dispositif portatif est un bracelet, une bague ou une boucle d'oreille.

13. Capteur micro-électronique selon l'une quelconque des revendications 8 à 10, dans lequel ledit capteur est connecté à une armature, ou un châssis, ou des éléments d'affichage sensibles capacitifs d'un téléphone intelligent, d'une montre intelligente ou d'un gadget électronique personnel, ledit téléphone intelligent, ladite montre intelligente ou ledit gadget électronique personnel étant capable d'une transduction de charge électrique au capteur.

14. Capteur micro-électronique selon l'une quelconque des revendications 8 à 10, dans lequel ledit capteur est un capteur d'empreintes digitales au sein d'un verrou de téléphone intelligent et ou d'un verrou de voiture, ou ledit capteur est intégré dans un module d'authentification biométrique d'un système de sécurité, d'un ordinateur personnel, d'un ordinateur portable, d'un système d'ouverture de portillon automatique, d'un système d'ouverture de portillon pivotant, d'une barrière de trappe, d'un tourniquet, ou d'une machine de caisse automatique, ou ledit capteur est intégré dans une puce d'authentification biométrique d'une carte de crédit ou d'une carte ou étiquette d'identification, ou ledit capteur est approprié pour une utilisation dans la surveillance hémodynamique vitale d'un conducteur dans le but de déterminer la somnolence, la fatigue, la nervosité, le risque cardiaque, le stress et d'autres situations dangereuses pour la conduite d'une voiture.

15. Procédé pour une authentification biométrique d'un utilisateur comprenant :
(1) la mise en contact d'un point de détection unique sur le corps de l'utilisateur avec le capteur micro-électronique de l'une quelconque des revendications 8 à 14, ou le positionnement à distance dudit capteur dans un espace contre le corps de l'utilisateur, et l'activation dudit capteur à la demande ou lorsque le contact est établi ;
(2) l'enregistrement de signaux électriques reçus depuis le corps de l'utilisateur avec ledit capteur micro-électronique sous une forme d'un courant électrique de source-drain du capteur en fonction du temps (dynamique ID_{S}) et/ou la mesure de paramètres S11-S12 du capteur en fonction du temps (dynamique S₁₁-S₁₂) ;
(3) la transmission des signaux enregistrés dudit capteur à une mémoire externe pour un traitement supplémentaire ; et
(4) la conversion des signaux transmis en signaux numériques, le traitement des signaux numériques dans la mémoire externe, et la comparaison de ladite dynamique I_{DS} et/ou de ladite dynamique S11-S12 à des données biométrique pré-étalonnées de l'utilisateur stockées dans la mémoire externe, pour ainsi authentifier biométriquement l'utilisateur.
